Europäisches Patentamt

European Patent Office

Office européen des brevets

(19)

(11) Publication number: **0 379 890**

**A1**

(12) **EUROPEAN PATENT APPLICATION**

(21) Application number: **90100457.2**

(22) Date of filing: **10.01.90**

(51) Int. Cl.⁵: **C12N 15/62, C12N 15/58, C12N 15/57, C12N 9/72, A61K 37/547**

The microorganism(s) has (have) been deposited with Fermentation Research Institute under number(s) FERM BP-2247 and FERM BP-2248.

(30) Priority: **23.01.89 GB 8901422**

(43) Date of publication of application: **01.08.90 Bulletin 90/31**

(84) Designated Contracting States: **AT BE CH DE DK ES FR GB GR IT LI LU NL SE**

(71) Applicant: **FUJISAWA PHARMACEUTICAL CO., LTD.**
**3, Doshomachi 4-chome Higashi-ku Osaka-shi Osaka 541(JP)**

(72) Inventor: **Niwa, Mineo**
**7-15, Kirinokuchi Kamiueno-cho Muko-shi Kyoto 617(JP)**
Inventor: **Satoh, Susumu**
**7-9-4, Higashitokiwadai Toyono-cho Toyono-gun Osaka 563-01(JP)**
Inventor: **Suzuki, Shingo**
**8-19, Misaki 7-chome Suminoe-ku Osaka-shi Osaka 559(JP)**
Inventor: **Otsuka, Kazuyuki**
**5-36-211, Nankounaka 5-chome Suminoe-ku Osaka-shi Osaka 559(JP)**
Inventor: **Kusunoki, Chihiro**
**6-B-407, Satsukigaokahigashi Suita-shi Osaka 565(JP)**

(74) Representative: **Bühling, Gerhard, Dipl.-Chem. et al**
**Patentanwaltsbüro Tiedtke-Bühling-Kinne Grupe-Pellmann-Grams-Struif-Winter-Roth Bavariaring 4 D-8000 München 2(DE)**

(54) **New tissue plasminogen activator.**

㉗ New tissue plasminogen activator which has a strong activity for converting plasminogen into plasmin and is useful as a thrombolytic agent comprising a
N-terminal peptide of plasminogen linked to t-PA having a special amino acid sequence;
a DNA encoding amino acid sequence of the t-PA as mentioned;
an expression vector comprising the said DNA and a pharmaceutical composition comprising the said t-PA;
the said t-PA is produced by culturing a host cell transformed with an expression vector comprising DNA encoding amino acid sequence of the said t-PA in a nutrient medium, and recovering the resultant t-PA from the cultured broth.

Fig.1-(1)
Construction of expression vectors of the new t-PA

Fig. 1-(2)

Fig. 1-(3)

# NEW TISSUE PLASMINOGEN ACTIVATOR

This invention relates to a new tissue plasminogen activator. More particularly, it relates to a new tissue plasminogen activator which has a strong activity for converting plasminogen into plasmin that degrades the fibrin network of blood clot to form soluble products and therefore is useful as a thrombolytic agent, to DNA sequence encoding amino acid sequence thereof, to a process for the production thereof and a pharmaceutical composition comprising the same.

The whole amino acid sequence and structure of a native human "tissue plasminogen activator" (hereinafter referred to as "t-PA"] and DNA sequence coding for it derived from a human melanoma cell (Bowes) have already been clarified by recombinant DNA technology [ Cf. Nature 301, 214 (1983) ] . The native t-PA is a single chain serine protease which is converted to a two-chain form, heavy chain and light chain, linked by single disulfide bond with plasmin. The light chain(L) is a protease domain and therefore contains the active-site of the enzyme. The heavy chain(H) has a finger domain (F), a growth factor domain (E) and two kringle domains (i.e. kringle 1 and kringle 2 domains; $K_1$ and $K_2$). Accordingly, the native t-PA is composed of five functional domains F, E, $K_1$, $K_2$ and L [ Cf.European Patent Application laid open No. 0196920 and Proc. Natl. Acad. Sci. USA 83, 4670 (1986) ] .

The native t- PA is unstable and has an extremely short half life (about 1 - 8 minutes depending on the species) in vivo. From the result of various studies on modification of the native t-PA for prolongation of the half life thereof, the inventors of this invention have succeeded in producing a new t-PA which is more stable in vivo than the native t-PA by combining N-terminal peptide of plasminogen with t-PA.

Accordingly, this invention provides a new t-PA corresponding to N-terminal peptide of plasminogen linked to t-PA.

Detailed explanation of the new t-PA is given in the followings.

The whole amino acid sequence and structure of a native human plasminogen have also been clarified by some prior literatures [e.g. Chemical Reviews 81, 432 (1981) and Blood 67, 1532 (1986)]. The native plasminogen is also a single chain glycoprotein which is composed of N-terminal peptide ($Glu^1$ to $Lys^{76}$ of the native plasminogen), five kringles ($Lys^{77}$ to $Arg^{560}$) and B chain of plasmin ($Val^{561}$ to $Asn^{791}$) The whole cDNA sequence of a native plasminogen has also been clarified by recombinant DNA technology and the amino acid sequence deduced from the cDNA is slightly different from that as mentioned above [Cf. FEBS LETTERS 213, 254 (1987)] .

Preferable N-terminal peptide of plasminogen may include whole or a part of N-terminal peptide of the native human plasminogen and modified form thereof (e.g. the whole N-terminal peptide linked to oligopeptide etc).

Preferable t-PA may include whole or a part of the native t-PA (e.g. the native t-PA, t-PA essentially consisting of finger, kringle 1 and kringle 2 domains of the heavy chain and the light chain of the native t-PA, t-PA essentially consisting of kringle 1 and kringle 2 domains of the heavy chain and the light chain of the native t-PA, t-PA essentially consisting of kringle 2 domain of the heavy chain and the light chain of the native t-PA, t-PA essentially consisting of the light chain of the native t-PA and t-PA essentially consisting of growth factor, kringle 1 and kringle 2 domains of the heavy chain and the light chain of the native t-PA, ) and a t-PA prepared by deletion or substitution of the constitutional amino acid(s) of the said t-PAs(e.g. the native t-PA in which $Arg^{275}$ is substituted with $Gly^{275}$, $Glu^{275}$ or $Asp^{275}$, etc.).

In this specification, numbering of constitutional amino acid of the native or new t-PA is based on that proposed by Pennica et. al.[Cf. Nature 301, 214 (1983)] and for example, $Tyr^2$ means Tyrosine at the position 2 of amino acid sequence of the native t-PA.

Preferable example of the new t-PA of this invention can be represented by the following amino acid sequences.

$A^1$ - $A^2$ - $A^3$     (I)

wherein $A^1$ is

SerGluProLeuAspAspTyrValAsnThrGlnGlyAlaSerLeuPheSerValThrLys

LysGlnLeuGlyAlaGlySerIleGluGluCysAlaAlaLysCysGluGluAspGluGlu
PheThrCysArgAlaPheGlnTyrHisSerLysGluGlnGluCysValIleMetAlaGlu
AsnArgLysSerSerIleIleArgMetArgAspValValLeuPheGluLysLysVal-,
↑

SerGluProLeuAspAspTyrValAsnThrGlnGlyAlaSerLeuPheSerValThrLys
LysGlnLeuGlyAlaGlySerIleGluGluCysAlaAlaLysCysGluGluAspGluGlu
PheThrCysArgAlaPheGlnTyrHisSerLysGluGlnGluCysValIleMetAlaGlu
AsnArgLysSerSerIleIleArgMetArgAspValValLeuPheGluLysLysValGlu
↑                                   ←.
Gly-,

SerGluProLeuAspAspTyrValAsnThrGlnGlyAlaSerLeuPheSerValThrLys
LysGlnLeuGlyAlaGlySerIleGluGluCysAlaAlaLysCysGluGluAspGluGlu
PheThrCysArgAlaPheGlnTyrHisSerLysGluGlnGlnCysValIleMetAlaGlu
AsnArgLysSerSerIleIleIleArgMetArgAspValValLeuPheGluLysLysVal- or

SerGluProLeuAspAspTyrValAsnThrGlnGlyAlaSerLeuPheSerValThrLys
LysGlnLeuGlyAlaGlySerIleGluGluCysAlaAlaLysCysGluGluAspGluGlu
PheThrCysArgAlaPheGlnTyrHisSerLysGluGlnGlnCysValIleMetAlaGlu
AsnArgLysSerSerIleIleIleArgMetArgAspValValLeuPheGluLysLysVal
GluGly-,

$A^2$ is
a bond or the same amino acid sequence as $Ser^1$ to $Val^{48}$ of the native t-PA , and
$A^3$ is
the same amino acid sequence as $Asp^{87}$ to $Pro^{527}$ of the native t-PA.

In this specification, the following code names for the new t-PA are conveniently employed.

plgΔGFTPA

The new t-PA of the formula (I) in which $A^1$ is
SerGluProLeuAspAspTyrValAsnThrGlnGlyAlaSerLeuPheSerValThrLys LysGln-
LeuGlyAlaGlySerIleGluGluCysAlaAlaLysCysGluGluAspGluGlu PheThrCysArgAlaPheGlnTyrHisSerLysGluGln-
GluCysValIllemetAlaGlu AsnArgLysSerSerIleIleArgMetArgAspValValLeuPheGluLysLysValGlu Gly-,
$A^2$ is
the same amino acid sequence as $Ser^1$ to $Val^{48}$ of the native t-PA, and
$A^3$ is
the same amino acid sequence as $Asp^{87}$ to $Pro^{527}$ of the native t-PA.

plgNTQTPA

The new t-PA of the formula (1) in which

A$^1$ is

SerGluProLeuAspAspTyrValAsnThrGlnGlyAlaSerLeuPheSerValThrLys LysGln-LeuGlyAlaGlySerIleGluGluCysAlaAlaLysCysGluGluAspGluGlu PheThrCysArgAlaPheGlnTyrHisSerLysGluGln-GluCysValIlemetAlaGlu AsnArgLysSerSerIleIleArgMetArgAspValValLeuPheGluLysLysVal-,

A$^2$ is

a bond, and

A$^3$ is

the same amino acid sequence:Asp$^{87}$ to Pro$^{527}$ of the native t-PA.

The new t-PA of this invention can be prepared by recombinant DNA technology and polypeptide synthesis.

Namely, the new t-PA can be prepared by culturing a host cell transformed (transfected) with an expression vector comprising DNA encoding amino acid sequence of the new t-PA in a nutrient medium and recovering from the cultured broth.

In this process, particulars of which are explained in more detail as follows.

The host cell may include microorganisms [bacteria (e.g. Escherichia coli, Bacillus subtilis, etc.), yeast (e.g. Saccharomyces cerevisiae, etc.), animal cell lines and cultured plant cells]. Preferred examples of the microorganism may include bacteria, especially a strain belonging to the genus Escherichia (e.g. E. coli HB101 ATCC 33694, E. coli HB101-16 FERM BP-1872, E. coli 294 ATCC 31446, etc.), yeast, especially a strain belonging to the genus Saccharomyces [e.g. Saccharomyces cerevisiae AH22 ], animal cell lines [e.g.mouse L929 cell, Chinese hamster ovary (CHO) cell etc.] and the like.

When bacterium ,especially E. coli is used as a host cell, the expression vector is usually composed of at least promoter-operator region, initiation codon, DNA encoding the amino acid sequence of the new t-PA, termination codon, terminator region and replicatable unit. When yeast or animal cells are used as host cells,the expression vector is preferably composed of at least promoter, initiation codon, DNA encoding the amino acid sequences of the signal peptide and the new t-PA and termination codon, and it is possible that enhancer sequence, 5$^{'}$-and 3$^{'}$-noncoding region of the native t-PA, splicing junctions, polyadenylation site and replicatable unit is also inserted into the expression vector.

The promoter-operator region comprises promoter, operator and Shine-Dalgarno (SD) sequence (e.g. AAGG, etc.). Preferable promoter-operator region may include conventionally employed promoter-operator region (e.g. PL-promoter and trp-promoter for E. coli). The promoter for expression of the new t-PA in yeast may include the promoter of the TRP1 gene, the ADHI or ADHII gene and acid phosphatase (pH05) gene for S. cerevisiae and the promoter for expression of the new t-PA in mammalian cells may include SV40 early or late-promoter, HTLV-LTR-promoter, mouse metallothionein I(MMT)-promoter, vaccinia-promoter and the like.

Preferable initiation codon may include methionine codon (ATG).

The signal peptide may include the signal peptide of the native t-PA, the signal peptide of the native plasminogen and the like.

The DNA encoding the amino acid sequence of the signal peptide or the new t-PA can be prepared in a conventional manner such as a partial or whole DNA synthesis using DNA synthesizer and/or treatment of the complete DNA sequence coding for the native or mutant t-PA inserted in a suitable vector [e.g. pTPA21, pTPA25, pTPA102, pTPA102 (Lys277→Ile), p51H, pN53] obtainable from a transformant [e.g. E. coli LE392λ$^{+}$(pTPA21), E. coli JA221 (pTPA25) ATCC 39808, E. coli JA221 (pTPA102) ATCC 39810, E. coli JA221 (pTPA102)(Lys 277→Ile) ATCC 39811, E. coli JM109(p51H) FERM P-9774, E. coli JM109(pN53) FEB P-9775] with a suitable enzyme (e.g. restriction enzyme, alkaline phosphatase, polynucleotide kinase, DNA ligase, DNA polymerase, etc.). In the preferred embodiment of this invention, the preparation of DNA encoding the new t-PA can be carried out by casset mutagenesis described in Wells et. al., Science 233, 659 -663 (1986), and/or oligonucleotide-directed mutagenesis described in Fritz, DNA Cloning I, 151 (1985), IRL PRESS.

The termination codon(s) may include a conventionally employed termination codon(e.g. TAG, TGA, etc.).

The terminator region may include natural or synthetic terminator (e.g. synthetic fd phage terminator, etc.).

The replicatable unit is a DNA compound having capable of replicating the whole DNA sequence belonging thereto in a host cell and may include natural plasmid, artificially modified plasmid (e.g. DNA fragment prepared from natural plasmid) and synthetic plasmid and preferable examples of the plasmid may include plasmid pBR322 or artificially modified thereof (DNA fragment obtained from a suitable restriction enzyme treatment of pBR322) for E. coli, yeast 2 μ plasmid or yeast chromosomal DNA for yeast, plasmid pRSVneo ATCC 37198, plasmid pSV2dhfr ATCC 37145, plasmid pdBPV-MMTneo ATCC 37224, plasmid pSV2neo ATCC 37149 for mammalian cells.

The enhancer sequence may include the enhancer sequence (72 b.p.) of SV40.

The polyadenylation site may include the polyadenylation site of SV40.

The splicing junction may include the splicing junction of SV40.

The promoter, initiation codon, DNA encoding the amino acid sequence of the new t-PA, termination codon(s) and terminator region can consecutively and circularly be linked with an adequate replicatable unit (plasmid) together, if desired, using an adequate DNA fragment(s) leg. linker, other restriction site, etc.) in a conventional manner (e.g. digestion with restriction enzyme, ligation using T4 DNA ligase) to give an expression vector. When mammalian cells are used as host cells, it is possible that enhancer sequence, promoter, 5′-noncoding region of the cDNA of the native t-PA, initiation codon, DNA encoding amino acid sequences of the signal peptide and the new t-PA, termination codon(s), 3′-noncoding region, splicing junctions and polyadenylation site are consecutively and circularly be liked with an adequate replicatable unit together in the above manner.

A host cell can be transformed (transfected) with the expression vector. Transformation (transfection) can be carried out in a conventional manner [e.g. Kushner method for E. coli, calcium phosphate method for mammalian cells, microinjection, etc.] to give a transformant(transfectant).

For the production of the new t-PA in the process of this invention, thus obtained transformant comprising the expression vector is cultured in an aqueous nutrient medium.

The nutrient medium may contain carbon source(s)(e.g. glucose, glycerine, mannitol, fructose, lactose, etc.) and inorganic or organic nitrogen source(s) (e.g. ammonium sulfate, ammonium chloride, hydrolysate of casein, yeast extract, polypeptone, bactotrypton, beef extract, etc.). If desired, other nutritious sources [e.g. inorganic salts (e.g. sodium or potassium biphosphate, dipotassium hydrogen phosphate, magnesium chloride, magnesium sulfate, calcium chloride), vitamins (e.g. vitamin $B_1$), antibiotics (e.g. ampicillin, kanamycin),etc.] may be added to the medium. For the culture of mammalian cells, Dulbecco's Modified Eagle's Minimum Essential Medium (DMEM ) supplemented with fetal calf serum and an antibiotic is often used.

The culture of the transformant (including transfectant) may usually be carried out at pH 5.5 -8.5 (preferably pH 7 -7.5) and 18 - 40°C (preferably 25 - 38°C) for 5 - 50 hours.

When thus produced new t-PA exists in the culture solution, culture filtrate (supernatant) is obtained by filtration or centrifugation of the cultured broth. From the culture filtrate, the new t-PA can be purified in a conventional manner as generally employed for the purification and isolation of natural or synthetic proteins (e.g. dialysis, gel filtration, affinity column chromatography using anti-t-PA monoclonal antibody, column chromatography on a suitable adsorbent, high performance liquid chromatography, etc.). When the produced new t-PA exists in periplasm and cytoplasm of the cultured transformant, the cells are collected by filtration and centrifugation, and the cell wall and/or cell membrane thereof are destroyed by, for example, treatment with super sonic waves and/or lysozyme to give debris. The debris can be dissolved in a suitable aqueous soltuion (e.g. 8M aqueous urea, 6M aqueous guanidium salts). From the solution, the new t-PA can be purified in a conventional manner as exemplified above.

It is necessary to refold the new t-PA produced in E. coli. The refolding can be carried out in a conventional manner.

The new t-PA of this invention is useful as a thrombolytic agent for the treatment of vascular diseases (e.g. myocardial infarction, stroke, heart attack, pulmonary embolism, etc.). The new t-PA of this invention in admixture with pharmaceutically acceptable carrier(s) can be parenterally to mammals including human being in a form of a pharmaceutical composition such as injection or infusion preparation.

The pharmaceutically acceptable carrier may include various organic or inorganic carrier materials conventionally used in the preparation of pharmaceutical composition comprising peptide or protein (e.g. serum albumin, etc.).

A dosage of the new t-PA of this invention is to be varied depending on various factors such as kind of diseases, weight and/or age of a patient, and further the kind of administration route. The optimal dosage of the new t-PA of this invention is usually selected from a dose range of 0.1 - 10 mg/kg/day by injection or by infusion. The total daily amount mentioned above may divisionally be given to the patient for several hours.

Brief explanation of the accompanying drawings is as follows.

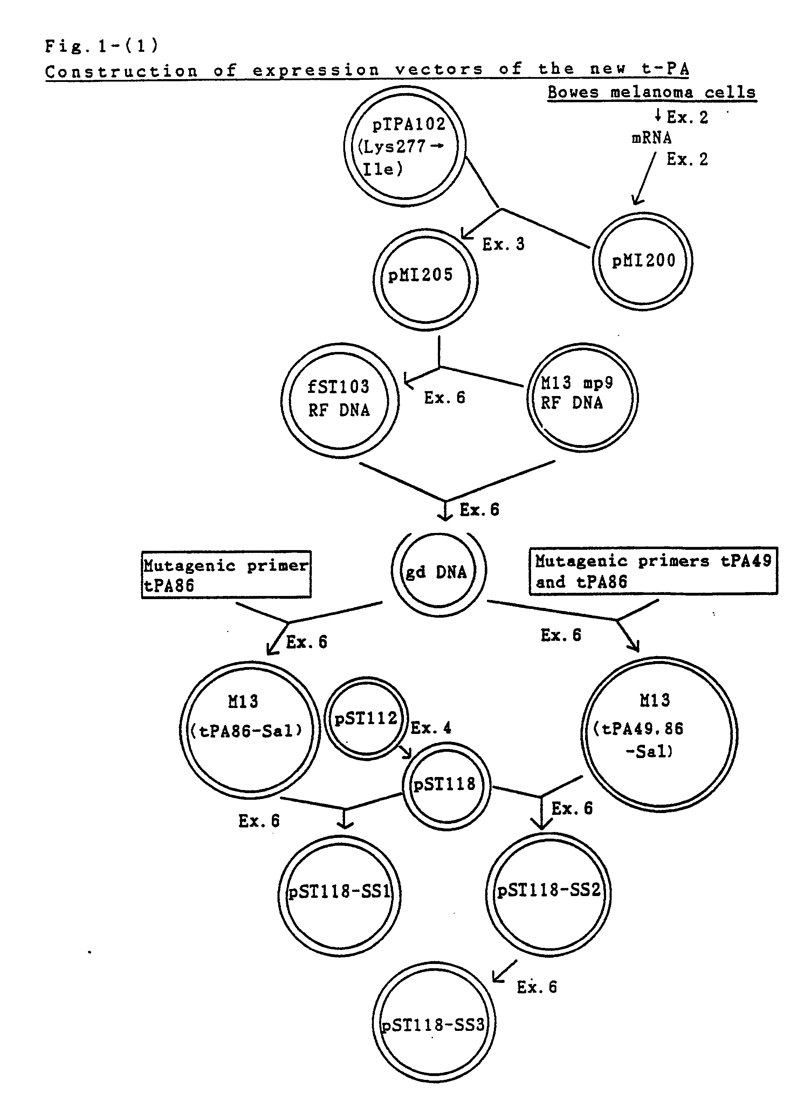

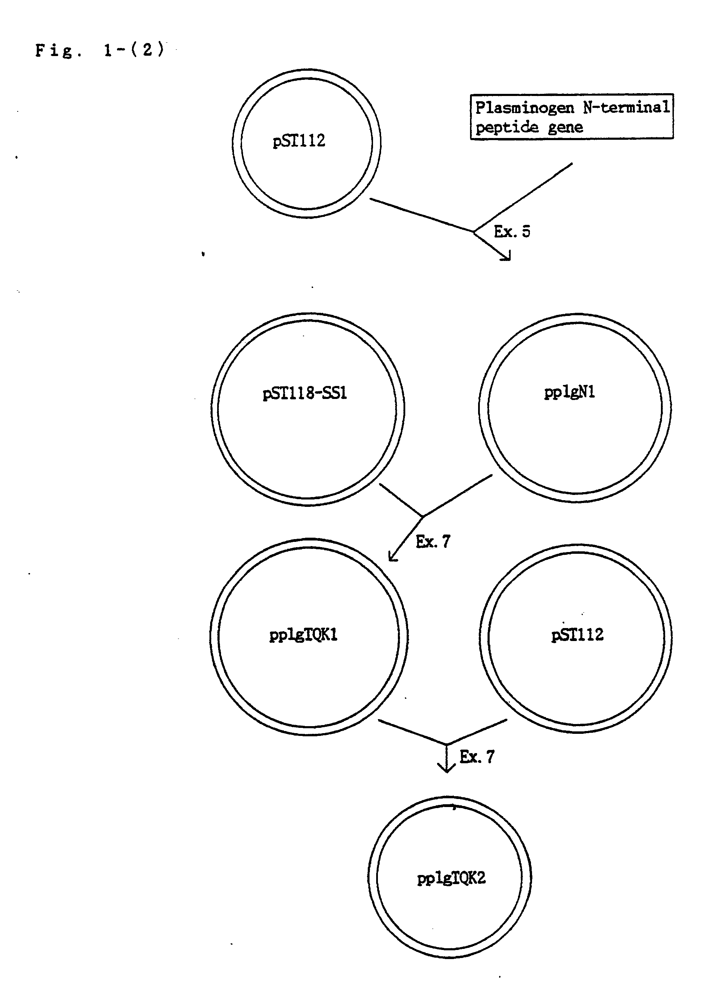

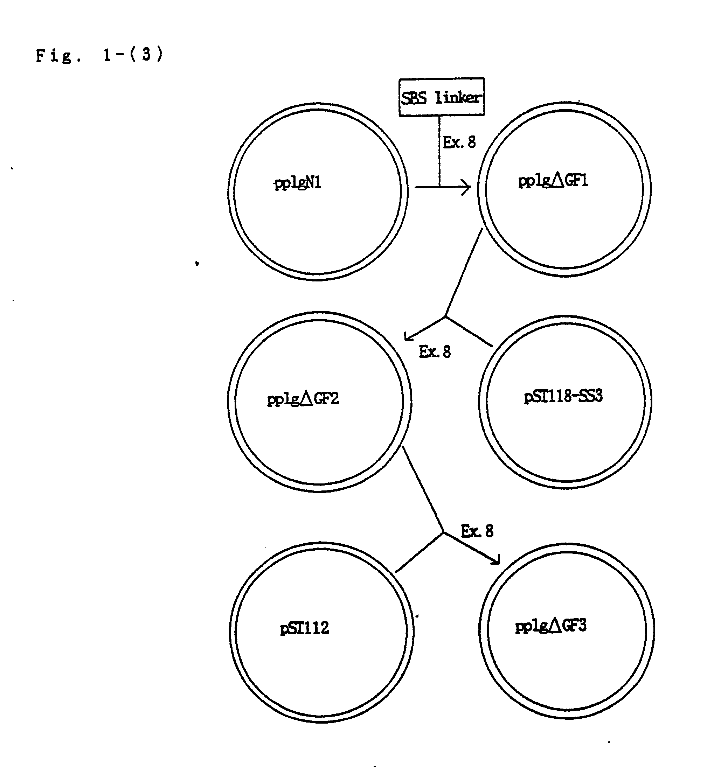

Figure 1 shows construction of expression vectors of the new t-PA .

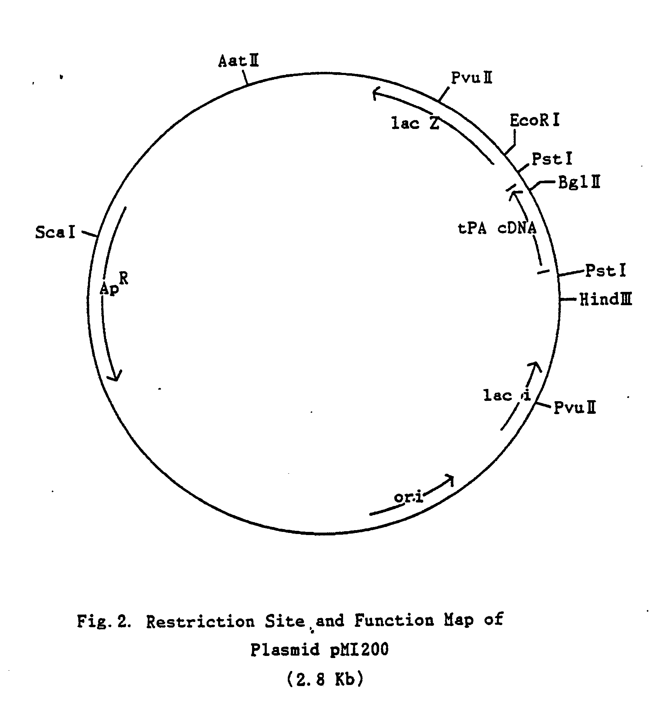

Figure 2 shows restriction site and function map of plasmid pMI200.

Figure 3 shows DNA sequence of the cDNA insert of plasmid pMI200.

Figure 4 shows DNA sequence of Bgl II DNA fragment (1974 bp) of plasmid pTPA102(Lys277→Ile).

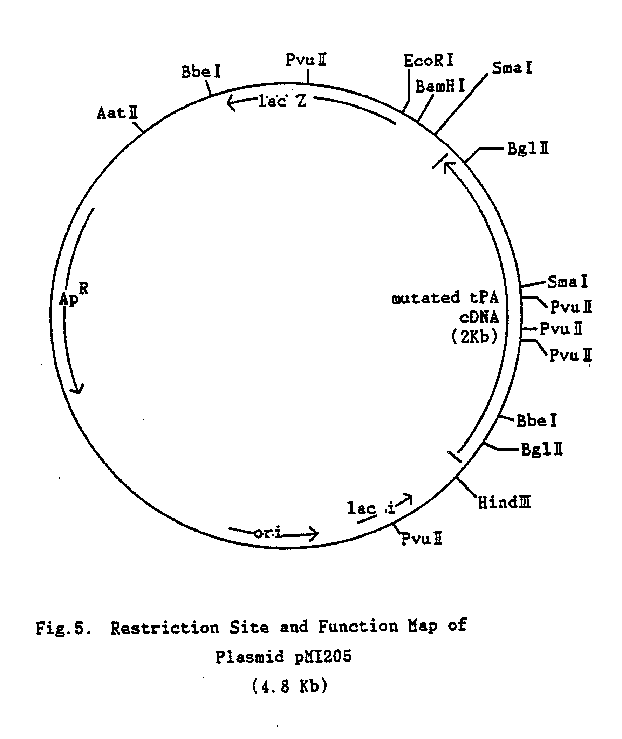

Figure 5 shows restriction site and function map of plasmid pMI205.

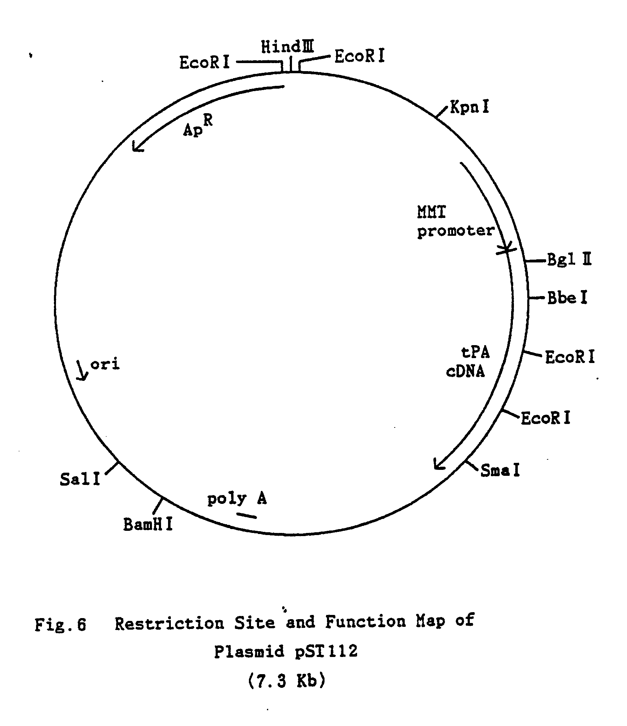

Figure 6 shows restriction site and function map of plasmid pST112.

Figure 7 shows cDNA sequence of a native tPA in pST112.

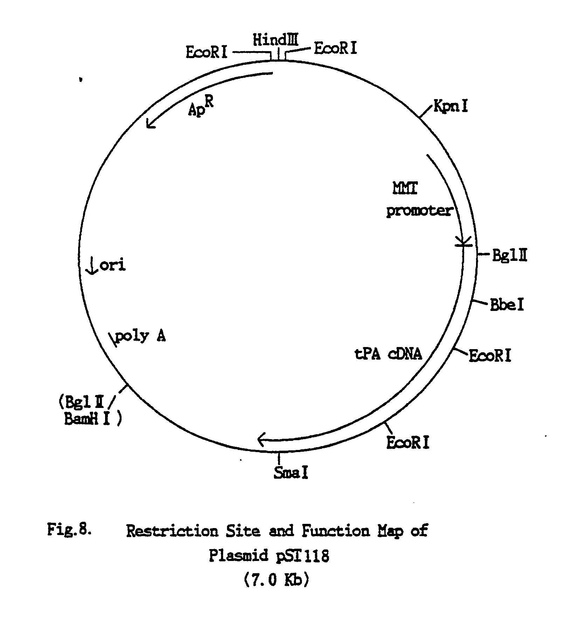

Figure 8 shows restriction site and function map of plasmid pST118.

Figure 9 shows construction of plasminogen N-terminal peptide gene. plasmid pplgN1.

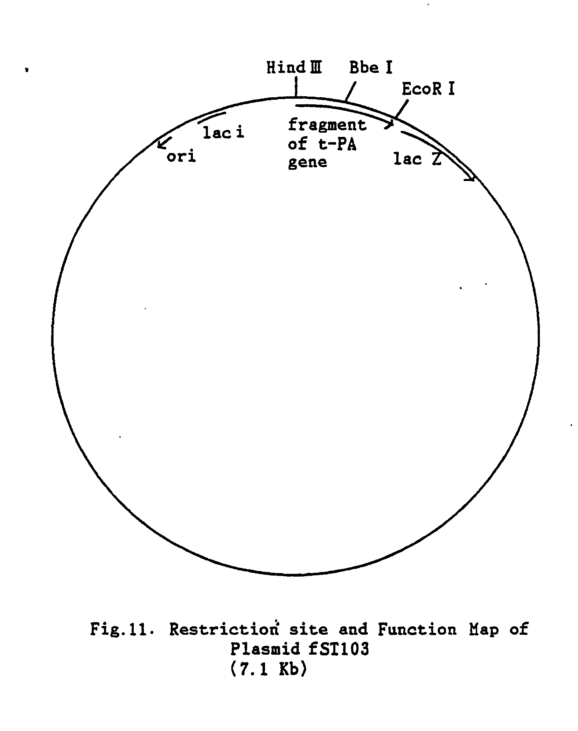

Figure 11 shows restriction site and function map of plasmid pST103.

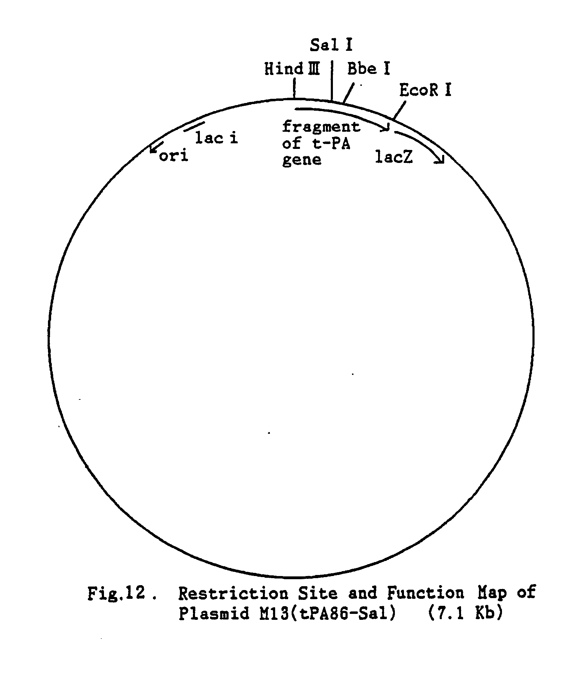

Figure 12 shows restriction site and function map of plasmid M13(tPA86-Sal).

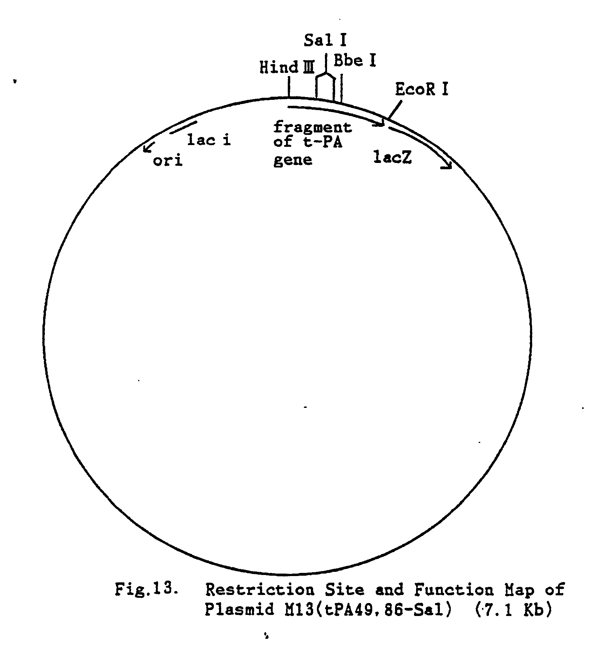

Figure 13 shows restriction site and function map of plasmid M13(tPA49,86-Sal).

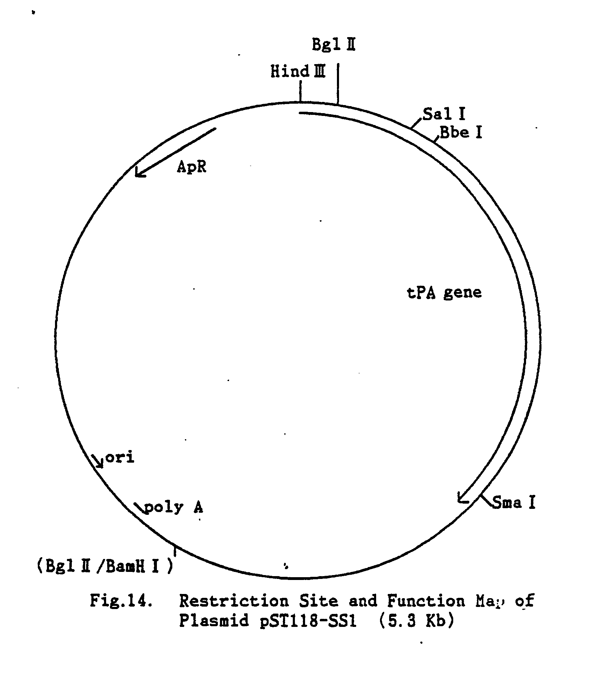

Figure 14 shows restriction site and function map of plasmid pST118-SS1.

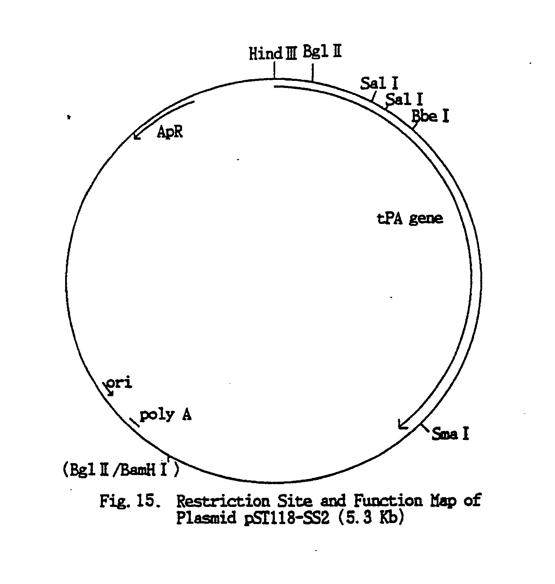

Figure 15 shows restriction site and function map of plasmid pST118-SS2.

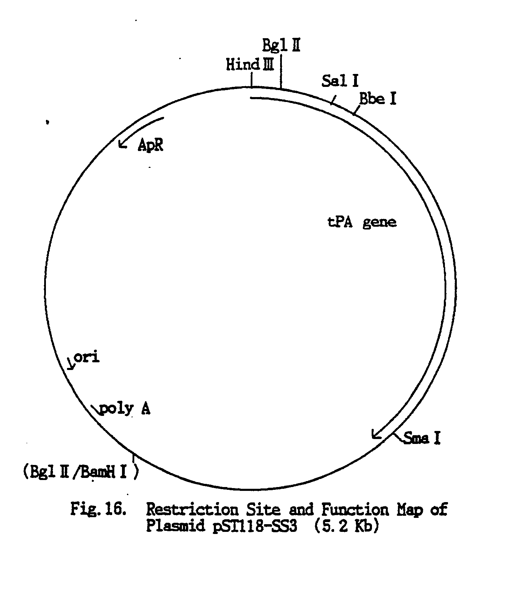

Figure 16 shows restriction site and function map of plasmid pST118-SS3.

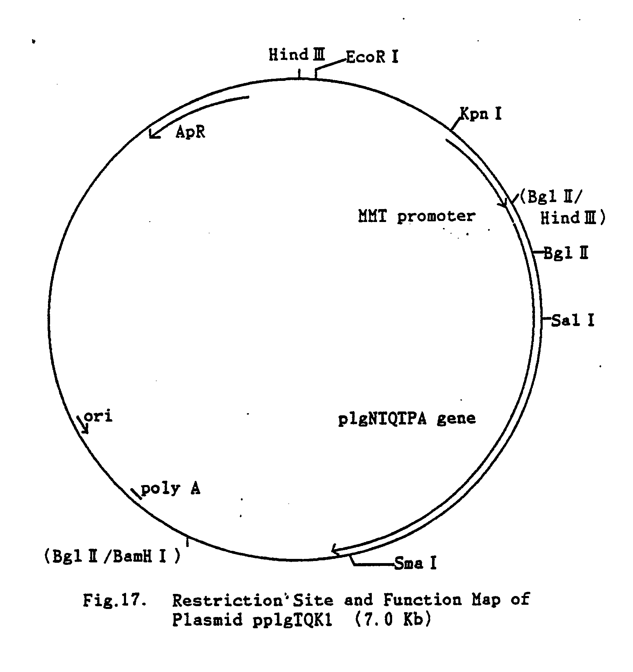

Figure 17 shows restriction site and function map of plasmid pplgTQK1.

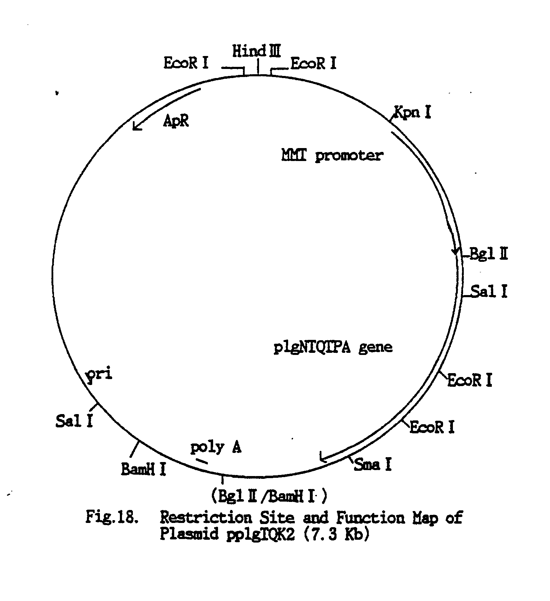

Figure 18 shows restriction site and function map of plasmid pplgTQK2.

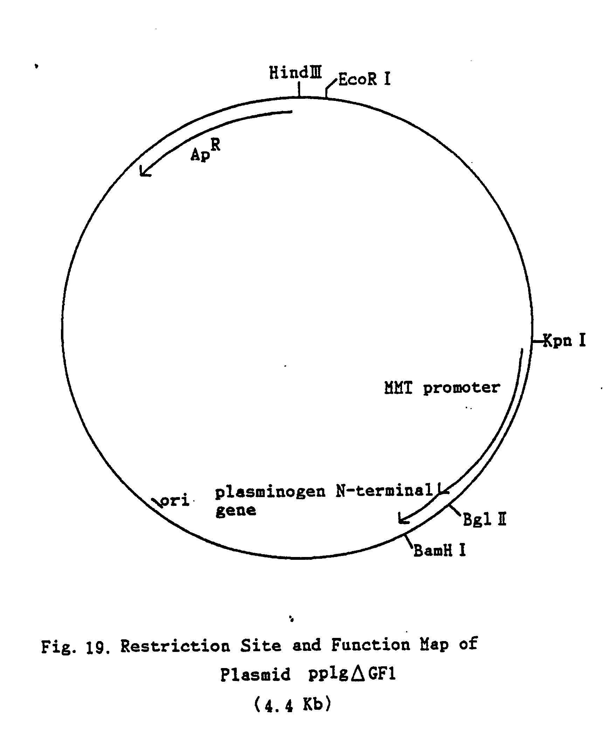

Figure 19 shows restriction site and function map of plasmid pplgΔGF1.

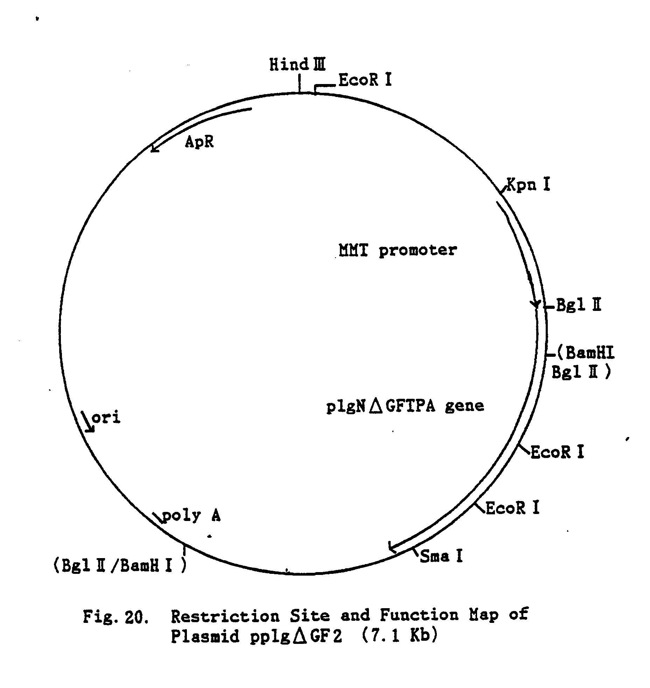

Figure 20 shows restriction site and function map of plasmid pplgΔGF2.

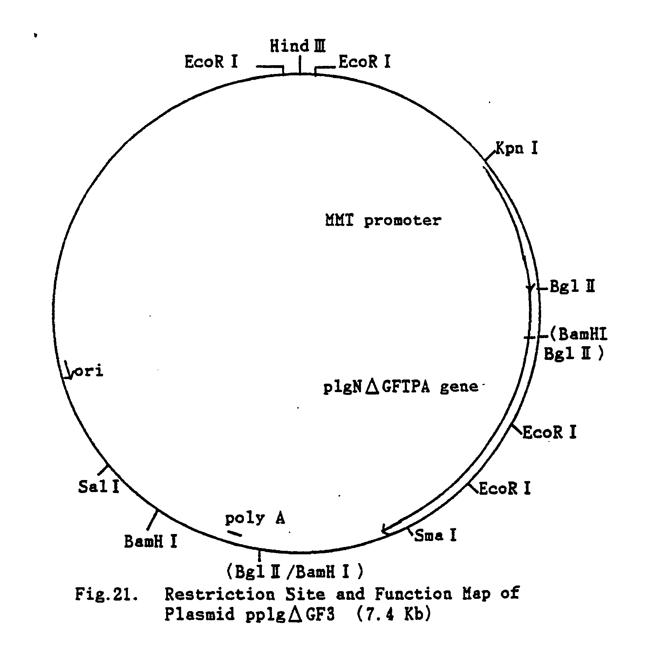

Figure 21 shows restriction site and function map of plasmid pplgΔGF3.

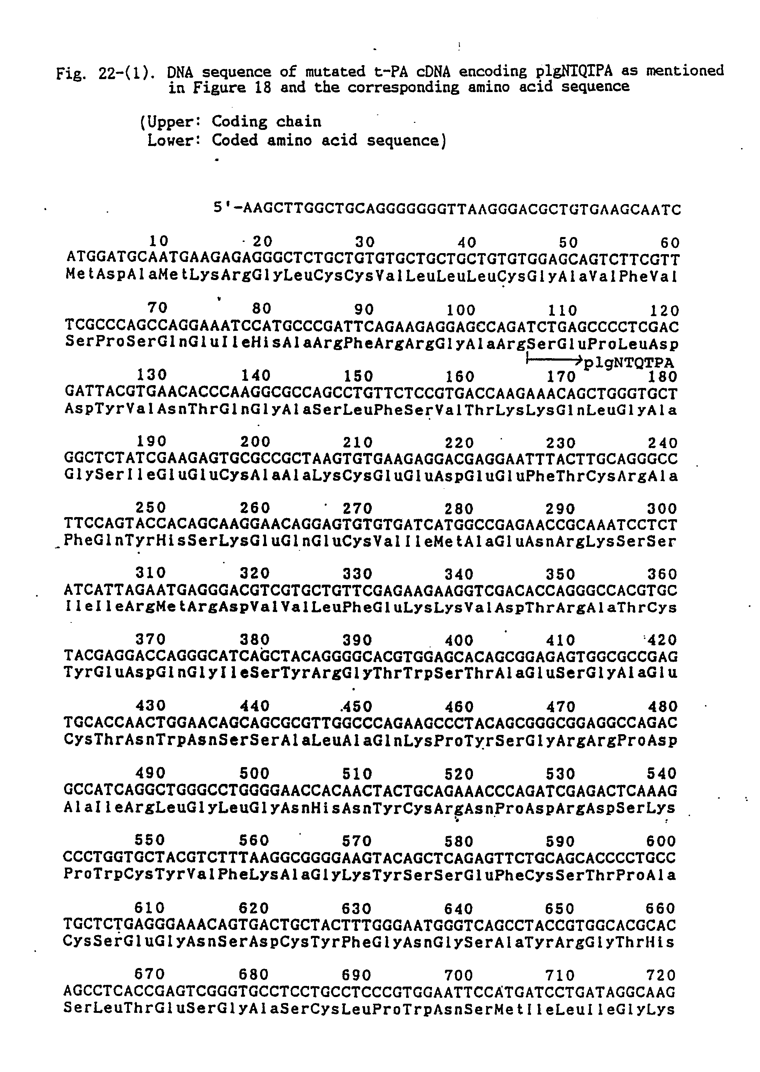

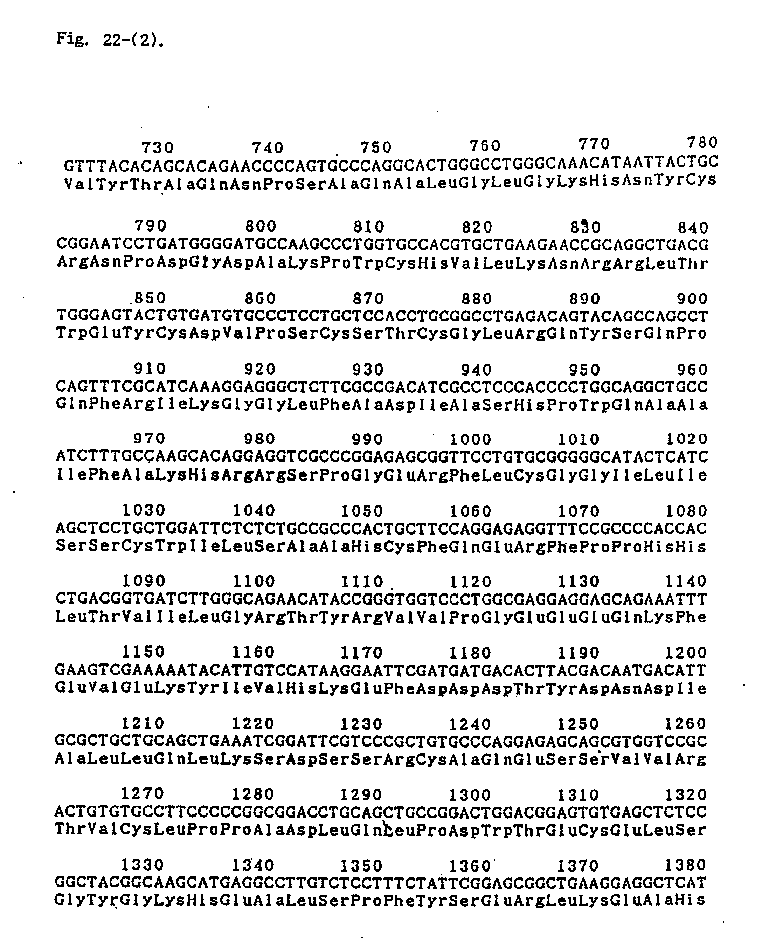

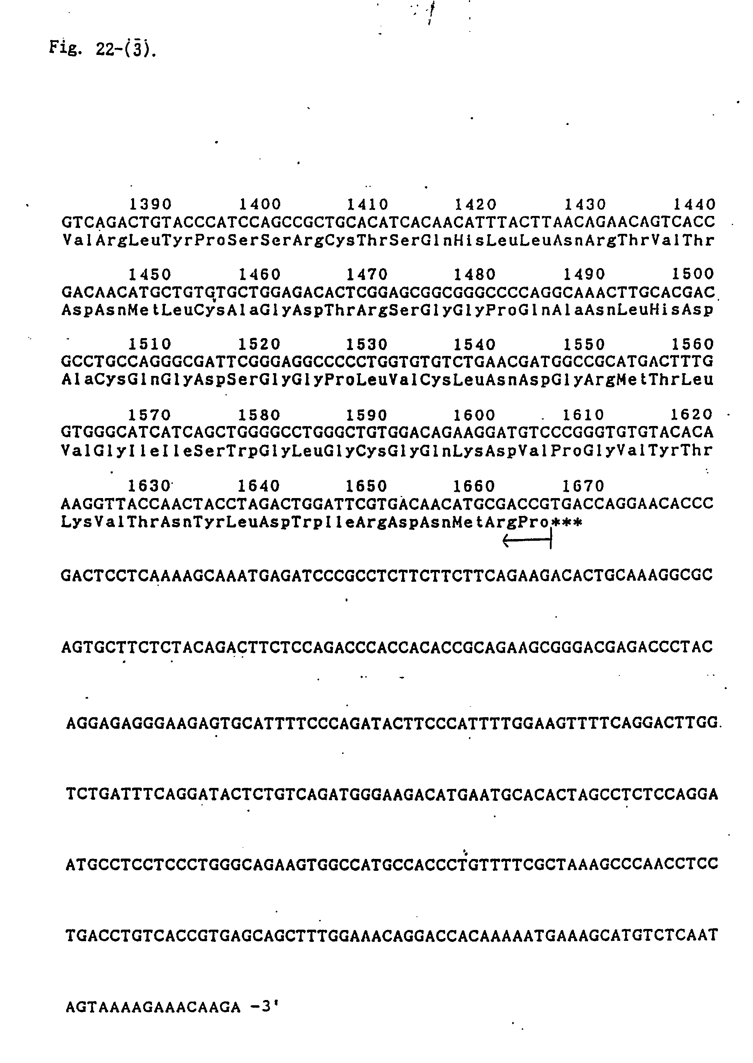

Figure 22 shows DNA sequence of mutated t-PA CDNA encoding plgNTQTPA as mentioned in Figure 18 and the corresponding amino acid sequence .

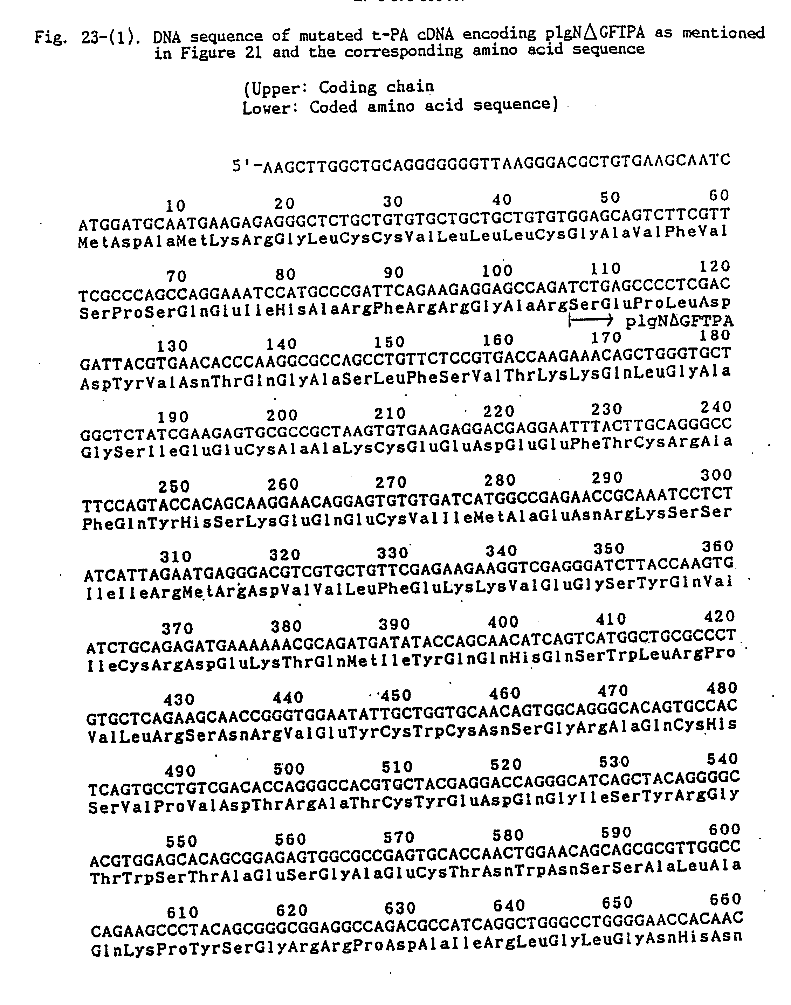

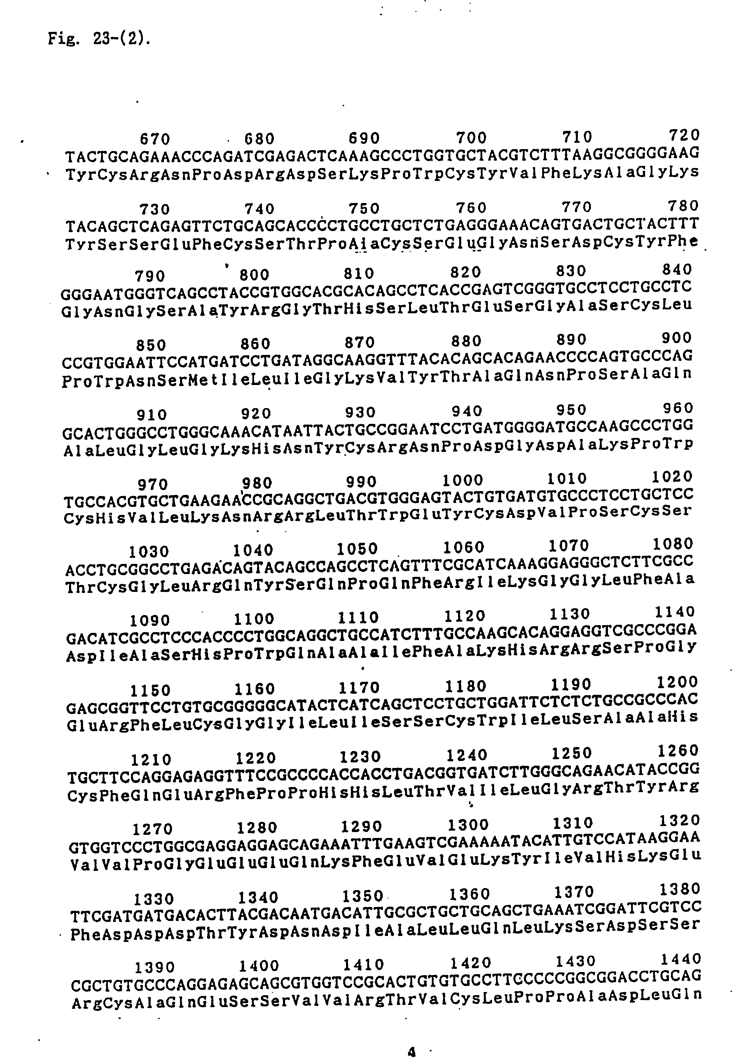

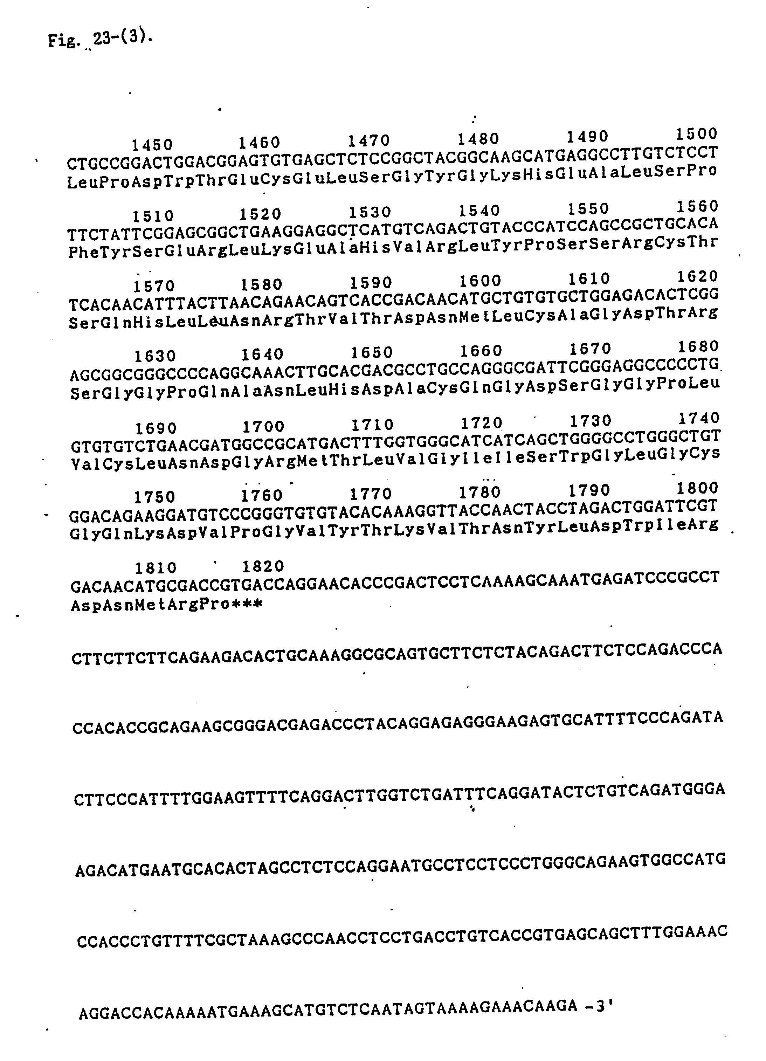

Figure 23 shows DNA sequence of mutated t-PA CDNA encoding plgNΔGFTPA as mentioned in Figure 21 and the corresponding amino acid sequence

The following Examples are given for the purpose of illustrating this invention in detail.

In the Examples, the following materials and general procedure were used.

A. Bacterial strains and cell lines

E. coli MM294 ATCC 31446, E. coli HB101 ATCC 33649, E. coli DH1 ATCC 33849 and E. coli JM 103 (available from Pharmacia) were used for bacterial transformation. Mouse L-929 cell ATCC CCL-1 was used as an expression cell lines.

B. Transformation and transfection

(1) E. coli was transformed under the following conditon.

A single fresh colony of E. coli was picked up and dispersed into 5 ml of sterile psi medium (5 g of Bacto Yeast Extract, 20 g of Bacto Tryptone, 5g of MgSO₄ per 1 ℓ of H₂O adjusted to pH 7.6 with KOH). After incubating at 37°C for 2 hours, 5 ml of the culture was inoculated into 100 ml of prewarmed and sterile psi medium. After incubating at 37°C for 2 hours, the culture broth was cooled at 0°C and centrifuged at 6, 000 rpm for 5 minutes at 4°C. The resultant pellet was gently resuspended in 40 ml of sterile Tfb I [30 mM KOAc, 10 mM RbCl, 10 mM CaCl₂, 50 mM MnCl₂, 15% (V/V) glycerol adjusted to pH 5.8]. The mixture was allowed to stand on ice for 5 minutes and then centrifuged at 6, 000 rpm for 5 minutes at 4°C. The resultant pellet was resuspended in 4 ml of sterile Tfb II [10 mM MOPS, 75 mM CaCl₂, 10 mM RbCl, 15% ((V/V) glycerol]. 200 μℓ of the suspension was poured into a microfuge test tube , frozen on dry ice and stored at -70°C. The frozen suspension was thawed at room temperature and then left on ice-water for 10 minutes. Up to 100 ng of plasmid DNA was added to the suspension and then on ice-water for 30 minutes. The mixture was heated at 42°C for 90 seconds and then returned to ice-water for 2 minutes. 800 μℓ of sterile psi medium was added to the mixture and incubated at 37°C for 1 hour. The mixture was spread on a proper selection medium plate (e.g. L broth containing 100 μg/ml ampicillin) for selection of the desired transformants. The plasmid obtained from the resultant transformant was confirmed by restriction enzyme analysis.

(2) Mammalian cell lines were transfected by a slightly modified method as that described in DNA cloning II, 152 (1985) IRL PRESS.

5 X 10⁵ mammalian cells were plated in a suitable medium [e.g.sterile Dulbecco's Modified Eagle's Essential Medium (DMEM) supplemented with 10%(V/V) fetal calf serum (FCS) and kanamycin] on a dish (diameter: 10 cm). After incubating at 37°C for 18 hours., the culture medium was replaced with fresh medium and the cells was incubated at 37°C for 3 hours.

On the other hand, the calcium phosphate-DNA mixture was prepared as follows.

i) the solution A: 1 ml of 2 x HEPES-buffered saline (10 g of HEPES, 1.6 g of NaCl, and 100 ml of distilled water ), and 20 $\mu\ell$ of 100 x phosphate buffer (70 mM Na$_2$HPO$_4$. 70 mM NaH$_2$PO$_4$).

ii) the solution B: 120 $\mu\ell$ of 2 M CaCl$_2$ solution and 30 $\mu$g of the purified plasmid DNA [if necessary, together with 0.3 $\mu$g of another plasmid containing suitable selection maker gene (e.g. pSV2neo ATCC 37149)] and 880 $\mu\ell$ of distilled water.

The solution B was added dropwise to the solution A with bubbling air. The mixture was allowed to stand at room temperature for 30 minutes and then gently pipetting.

The calcium phosphate-DNA mixture was added dropwise to the culture broth on the dish and then the cells were incubated at 37°C for 1 day. The culture medium was replaced with the suitable selection medium (e.g. medium containing antibiotic G418) and the cells were incubated. A level of t-PA production by the resultant clones were measured by an adequate assay (e.g. conventional indirect spectrophotomeric assay for t-PA).

C. Enzymes, Substrates and Plasmids

Restiriction endonucleases were purchased from New England Biolabs, Toyobo and Nippon Gene. T4 DNA ligase, T4 polynucleotide kinase, T4 DNA polymerase I , RNase H and bacterial alkaline phosphatase (BAP) were purchased from Takara Syuzo. DNA polymerase I Klenow fragment was purchased from Bethesda Research Laboratory. Reverse transcriptase (AMV) was purchased from Life Science Inc.. Calf intestinal alkaline phospatase (CIP) and terminal deoxynucleotidyl transferase was purchased from Pharmacia. These enzymes were used in a conventional manner (e.g. referring to a prescription attached to the commercially sold enzymes).

Human plasminogen and fibrinogen were purchased from The Green Cross. Bovine thrombin were purchased from Mochida Pharmaceutical. Antibiotic G418 was purchased from Sigma.

The plasmids, pSV2neo, pTPA102(Lys277→Ile) and pST112 can be isolated from E. coli transformants, ATCC 37149, ATCC 39811 and FERM BP-1966, respectively.

D. DNA preparation and other DNA cloning methods

Plasmid DNAs were prepared by the Alkaline method [Cf. Birnboim, H.C. Nucleic Acid Research 7, 1513 (1979)] and were purified by Sepharose CL-4B (Pharmacia) column chromatography for transfection use. DNA fragments obtained by restriction endonuclease digestion were isolated by electroelution from 0.8% agarose gel-followed by phenol-chloroform extraction and ethanol precipitation. Oligonucleotides were prepared by Model 381 DNA synthesizer (Applied Biosystems Inc.). Other cloning methods were made reference to Molecular Cloning [Maniatis, T. et al, Molecular Cloning, Cold Spring Harbor Laboratory (1982)] and DNA Cloning [Glover,D.M. ,DNA cloning Vol. I and II, IRL PRESS (1985)].

Example 1

Isolation of the human t-PA gene

A human genomic library containing partial digest of human DNA cloned into bacteriophage lambda Charon 4A at the EcoR I sites [available from T. Maniatis, Cf. Maniatis et. al., Cell 15, 687-701 (1978)] was screened with $^{32}$P nick-translated DNA probe by plaque hybridization [Cf. Wahl et. al., Proc. Natl. Acad. Sci. USA 76, 3683-3687 (1979)]. By using the 232-bp Pst I -Rsa I fragment of plasmid pTPA102 (Lys277→Ile) [an expression vector for a mutant t-PA (Lys277→Ile) which can be isolated from a transformant comprising the same, E. coli JA 221 (pTPA102) (Lys277→Ile) ATCC 39811. Cf. PCT International Publication No. 4086/01538 ] as a probe, three overlapping recombinants, λ18A1, λ19A2 and λ21A2 were isolated from a screening of 6 x 10⁶ plaques. To isolate a phage containing the transcriptional initiation site and 5′ -noncoding sequence of the t-PA gene, a second plaque hybridization was performed using a 1.5-kb EcoR I -BamH1 fragment from λ19A2 which includes 5′-noncoding sequence of the t-PA gene as a probe. This hybridization gave λ132A1 and λ110A1 which contain the first two exons of the t-PA gene. The inserted DNA fragments were mapped by restriction enzyme analysis and their DNA sequences were partially determined by the dideoxynucleotides chain termination method using M13 sequencing kit (Amersham). The sequence and mapping of their inserted DNA were found to be identical with the published t-PA genomic clone [Cf. Degen et. al., J. Biol. Chem. 261, 6972-6985 (1986)].

Example 2

Construction of plasmid pMI200

A. Isolation of t-PA CDNA clone containing the 5'-noncoding and signal sequences.

Total RNA from melanoma cell cultures was extracted in substantially the same manner as reported by Chirgwin et. al., Biochemistry 18, 5294 (1979). Human melanoma cells (Bowes) were cultured to confluent monolayers in ten rollar bottles, each filled in 400 ml of Dulbecco's modified Eagle's minimum essential medium (DMEM) containing 3% (vol/vol) fetal calf serum and 10 mM HEPES. To produce t-PA actively, the human melanoma cells were cultured in each 400 ml of DMEM containing 666 μg of the protease inhibitor aprotinin for 18 hours. The cells were collected by centrifugation and then resuspended in 50 ml of 6 M guanidinium isothiocyanate, 5 mM sodium citrate (pH 7.0), 0.1 M β-mercaptoethanol and 0.5% N-lauroylsarcosine sodium. The cells were dispersed by homogenization. The homogenate was layered onto 3 ml cushion of 5.7 M cesium chloride in 0.1 M EDTA (pH 7.5) in a Beckman SW 50.1 polyallomer tube and centrifuged at 30,000 rpm for 12 hours at 20 °C. The supernatant was discarded and the pellet of RNA was dissolved in 1 ml of water. The RNA was precipitated by addition of 0.1 volume of 3 M sodium acetate (pH 5.2) and 2.5 volumes of ethanol. Oligo-dT cellulose column chromatography was used to purify MRNA from the total RNA preparations [Cf. Aviv et. al., Proc. Natl. Acad. Sci. 69, 1408 (1972)]. Typical yields from 4 x $10^9$ cells were about 11 mg of total RNA and about 900 μg of poly(A)RNA.

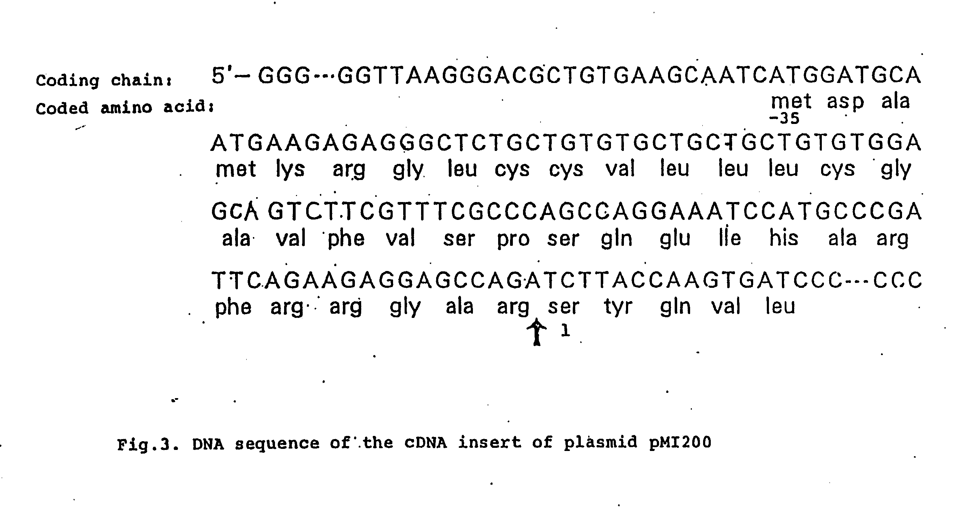

The poly(A)RNA was used for the preparation of double stranded cDNA by 5'-primer-extension method [Cf. Lawn et. al., Nucleic Acids Res.9, 6103-6114 (1981)]. In order to anneal poly(A)RNA with primer, 180 μg of poly(A)RNA and 5 μg of a primer having the sequence 5'-GATCACTTGGTAAGA-3', were heated in 200 μl of 0.1M XCl at 90 °C for 5 minutes and then slowly cooled to 42 °C. Using the annealed poly(A)-RNA, a first CDNA strand was synthesized with 200 units of reverse transcriptase in 400 μl of 50 mM Tris-HCl (pH 8.3), 10 mM MgCl₂, 10 mM DTT, 4 mM sodium pyrophosphate and 1 mM each four deoxyribonucleotides triphosphate (dATP, TTP, dCTP , dGTP) at 42 °C for 60 minutes. The mixture was treated with 20 units of RNase H, 100 units of DNA polymerase I and 100 units of E. coli DNA ligase in 2 ml of 20 mM Tris-KCl (pH 7.5), 5 mM MgCl₂, 10 mM (NH₄)₂SO₄, 100 mM KCl, 0.16 mM NAD and 50 μg/ml BSA at 12 °C for 1 hour and 22 °C for 1 hour. 15 U of 74 DNA polymerase was added to the reaction mixture. After incubating for 10 minutes at 37 °C, the reaction mixture was extracted once with phenol/chloroform (1/1) and the aqueous solution was precipitated with 1 volume of 4 M ammonium acetate and 4 volumes of ethanol. The double stranded cDNA was extended with deoxy (C) residues using 10 units of terminal deoxynucleotidyl transferase in 200 μl of 200mM potassium cacodylate, 25 mM Tris-HCl(pH6.9), 1 mM dCTP, 0.1 mM DTT, and 1 mM CoCl₂ at 37 °C for 30 minutes. The reaction mixture was extracted with phenol/chloroform and the aqueous solution was precipitated with 0.1 volume of 3 M sodium acetate (pH 5.2) and 2 volume of ethanol. The tailed cDNA was annealed with 660 ng of deoxy(G) tailed PUC 9 (Pharmacia) in 600 μl of 10 mM Tris-HCl (pH 8.0), 1 mM EDTA and 0.15 M NaCl at 58 °C for 1 hour. E. coli DH1 was transformed with the annealed DNA. Approximately 17, 000 transformants were obtained as ampicillin-resistant phenotype. The 5'-primer-extended cDNA library was screened with [32P] nick-translated DNA probe by colony hybridization [Cf. Grunstein et. al., Proc. Nat. Acad. Sci. 72, 396-3965 (1975)]. By using the 1.5-Kb EcoR I -BamHI fragment from λ19A2 as a probe, plasmid pMI200 containing the 5'-noncoding sequence and the signal sequence of t-PA cDNA was isolated from one of 8 colonies that gave a positive hybridization signal. The cDNA insert of pMI200 is about 170 bp long,which was sequenced by the method of the dideoxynucleotide chain termination (The cDNA sequence of which is shown in Fig.3).

Example 3

### Construction of plasmid pMI205

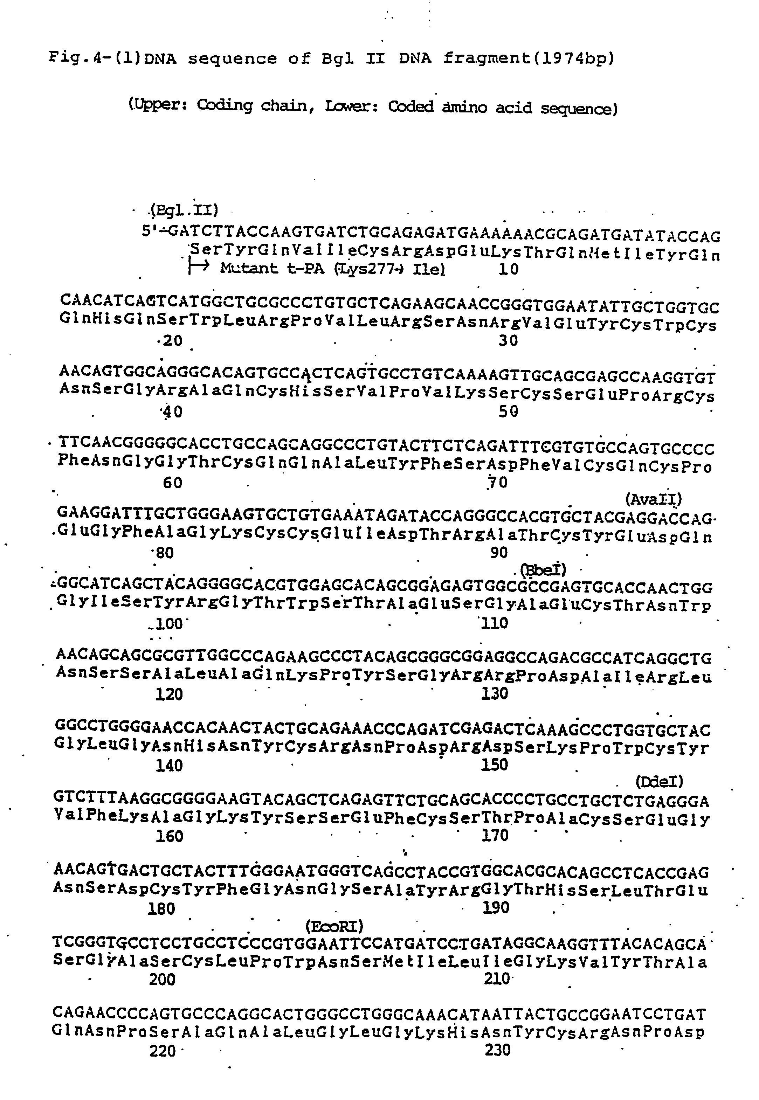

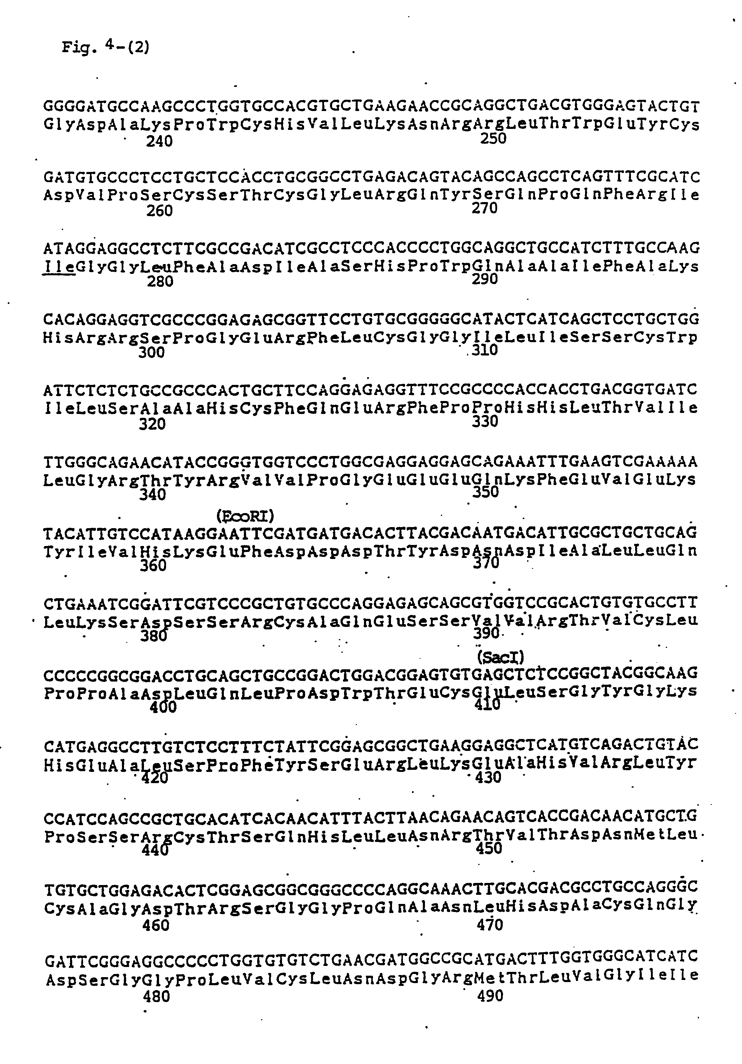

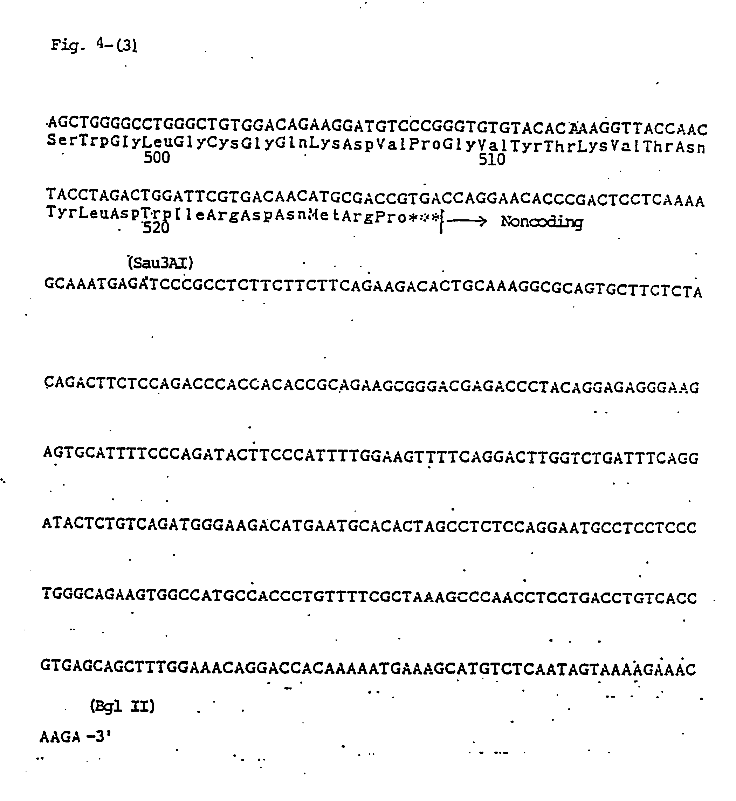

The plasmid pTPA 102 (Lys277→Ile) were digested with Bgl II. The 1974 bp Bgl II fragment (DNA sequence of which is illustrated in Fig.4) was isolated.

The plasmid pMI200 was digested with Bgl II. The linearized plasmid was treated with alkaline phospahtase. The DNA was extracted with a mixture of phenol and chloroform (1:1) and a mixture of chloroform and isoamyl alcohol (24:1), precipitated with ethanol and resuspended in water. The linealized and dephosphorylated plasmid pMI200 was and the 1974 bp Bgl II fragment of plasmid pTPA102(Lys277→Ile) were ligated with T4 DNA ligase.

E. coli DH1 was transformed with the ligation mixture and the resulting E. coli DH1 (pMI205) transformants were identified by their ampicillin-resistant phenotype and by several restriction enzyme analysis of their plasmid DNA.

### Example 4

### Construction of Plasmid pST118

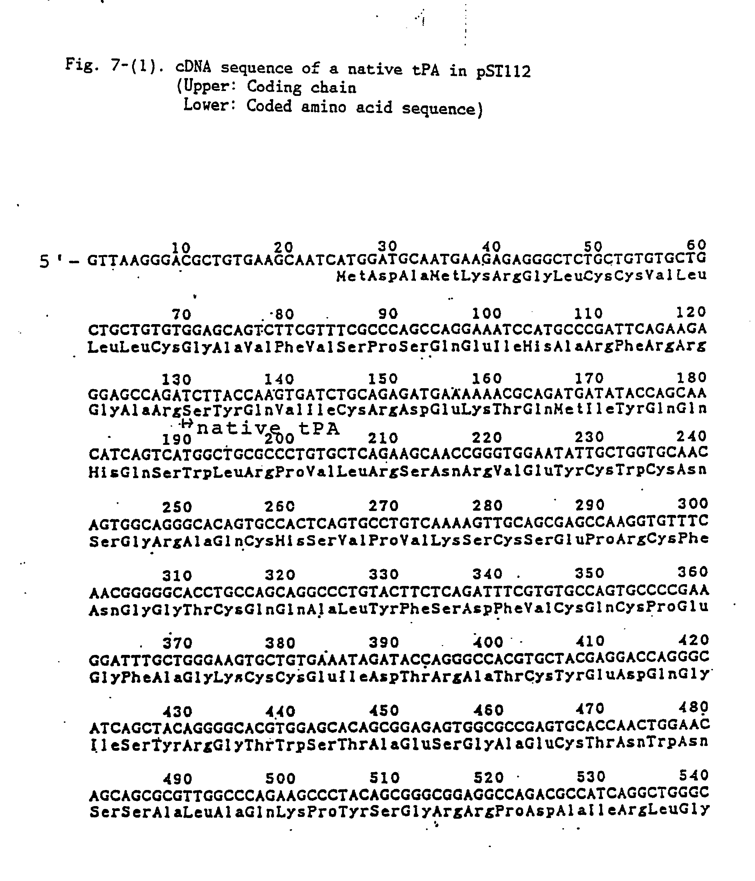

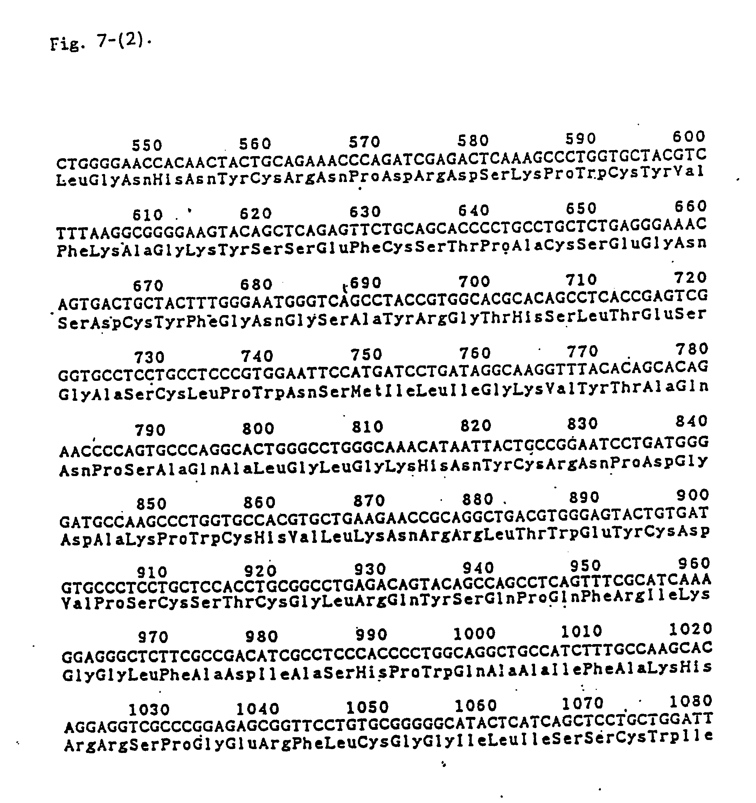

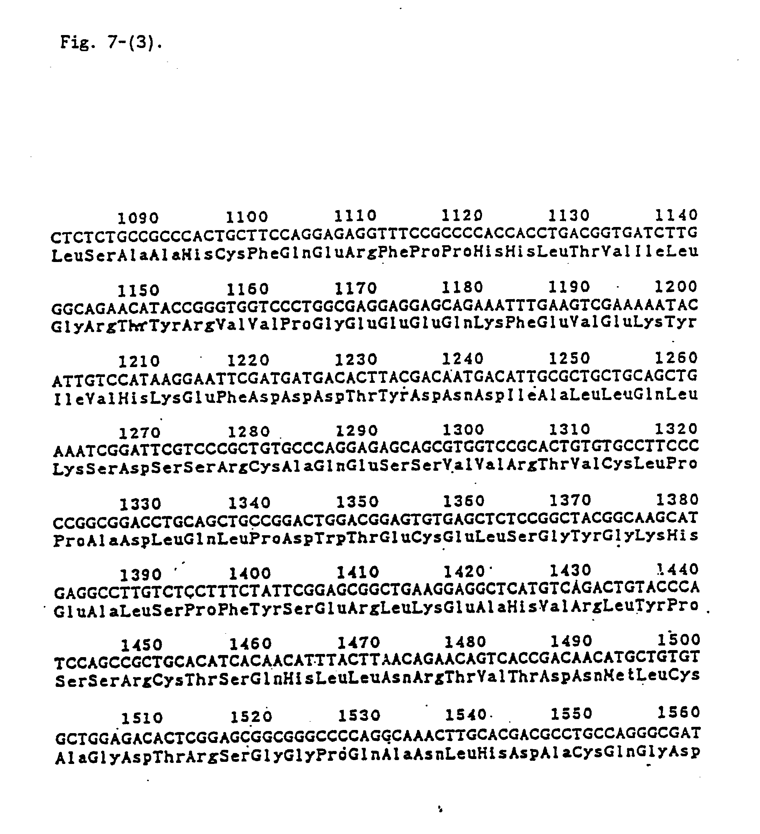

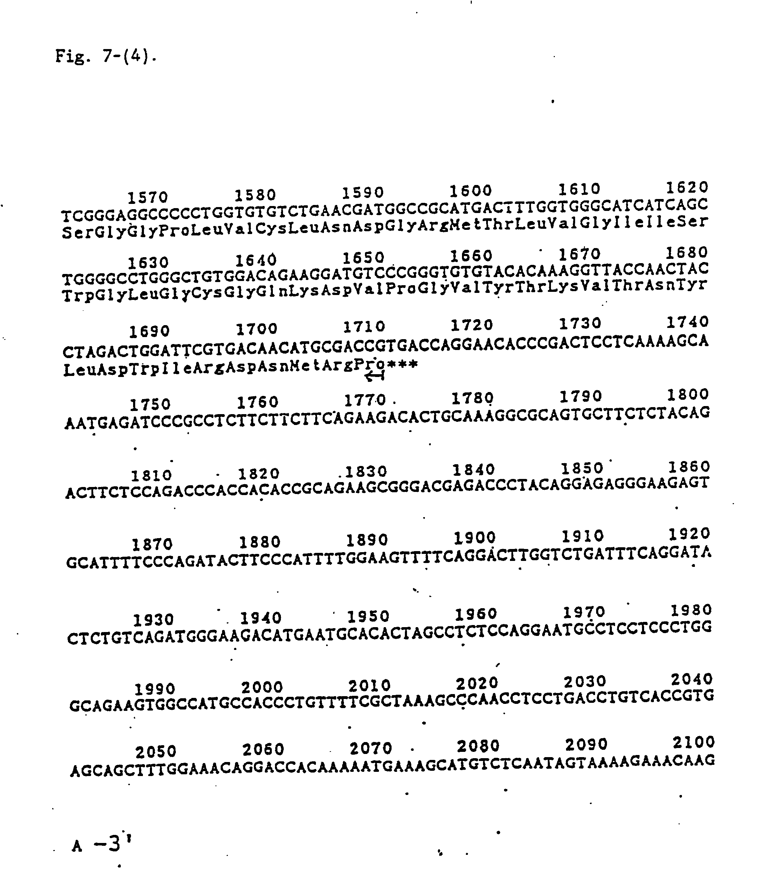

The plasmid pST112 (an expression vector for a native t-PA which can be isolated from a transformant comprising the same, E. coli DH1 FERM BP-1966, the complete cDNA sequence of a native tPA in pST112 is illustrated in Fig.7) were double-digested to completion with each of BamHI and Sal I . After phenol-chloroform (1:1) extraction, the DNA was precipitated with ethanol. BamHI-Sal I fragment was treated with DNA polymerase I Klenow-fragment. The DNA was recovered by ethanol precipitation. The resultant DNA were self-ligated. The ligation mixture was used to transform E. coli HB101. Transformants were identified by their ampicillin resistant phenotype and restriction enzyme analysis of plasmid DNA. Plasmid pST118 was isolated from one of the 9 transformants.

### Example 5

### Construction of Plasmid pplgN1

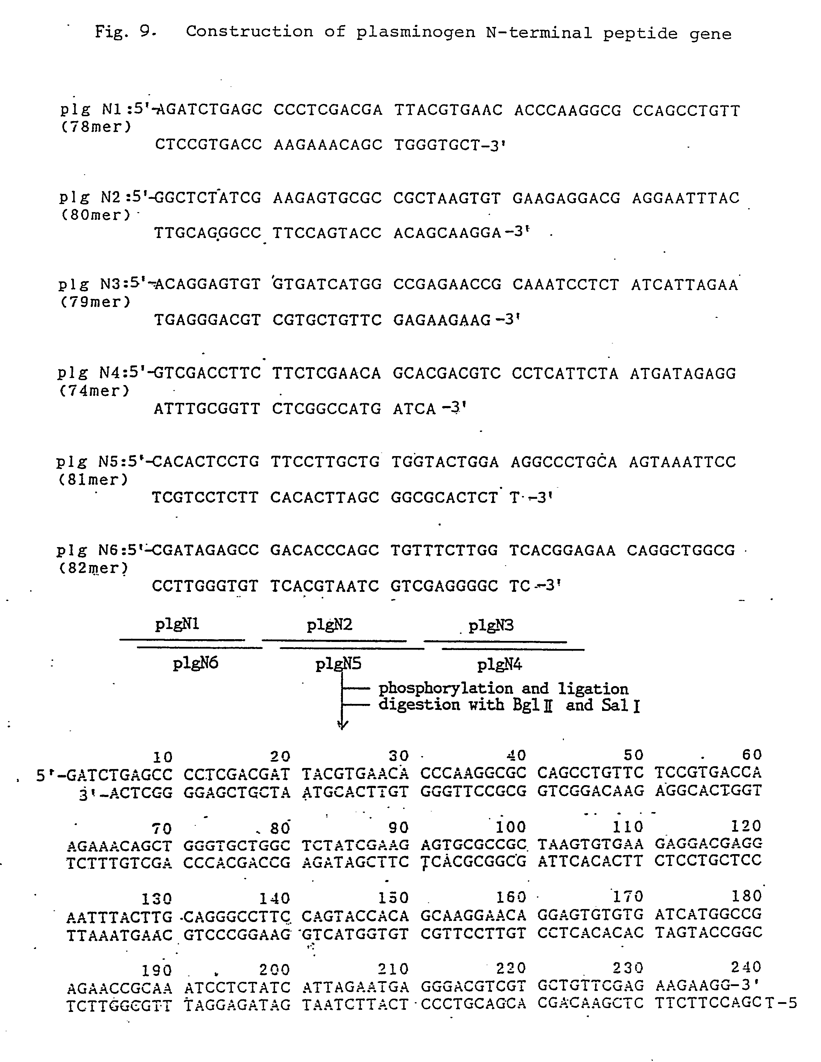

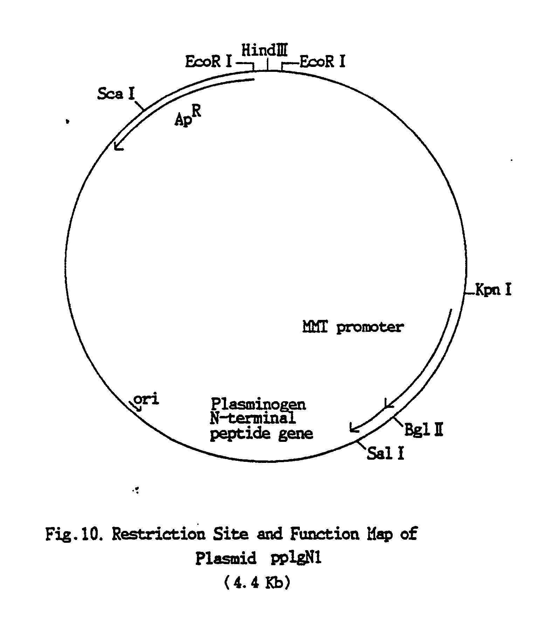

Human plasminogen N-terminal peptide gene was deduced from plasminogen peptide sequence [Cf. Chemical Reviews 81, 432 (1981) and Blood 67, 1532 (1986)] and was prepared to ligate with synthesized oligonucleotides, plg N1, plg N2, plg N3, plg N4, plg N5 and plg N6 as shown in Fig.9. 5 μg of each oligonucleotides were phosphorylated with T4 polynucleotides kinase and then ligated with T4 DNA ligase. The ligation mixture was digested with Bgl II and Sal I, and the resultant plasminogen N-terminal peptide gene (237 bp) was isolated by electroelution from 2.25% agarose gel. On the other hand, plasmid PST112 was digested with Bgl II and Sal I and about 4 kbp of Bgl II-Sal I fragment was isolated. These fragments were ligated with T4 DNA ligase and then E. coli MM294 was transformed with the ligation mixture. Transformants were identified by their ampicillin resistant phenotype and restriction enzyme analysis of plasmid DNA. Plasmid pplgN1 was isolated from one of the transformants.

### Example 6

### Construction of pST118-SS1, pST118-SS2 and pST118-SS3

To construct deletion mutant of the t-PA (e.g. finger domain lacking, growth factor domain lacking and /or kringle domain lacking t-PAs, etc.). Most convenient method is in vitro site specific mutagenesis [Cf. Zoller, M.J. and Smith, M., Method in Enzymology - Recombinsnt DNA, part B, 100, 468 (1983)]. By the method, proper restriction endonuclease sites were introduced in the specific site of genes. In this manner, finger and growth factor domains lacking t-PA gene and growth factor domain lacking t-PA gene were prepared.

The Hind III-EcoR I DNA fragment (0.76 kbp) of plasmid pMI205 was cloned in M13 mpg RF DNA [Cf.

Messing J., Method in Enzymology - Recombinant DNA, part B, 101, 20 (1983)] at the Hind III-EcoR I site. The resultant phage RF DNA(fST103) contains DNA encoding signal peptide to kringle 1 of the native t-PA. E. coli JM103 was transfected with the resulting phage DNA (fST103) and then the transfectant was cultivated at 37°C for 16 hours. The single strand DNA (ssDNA) was prepared from the culture supernatant in a conventional manner. The Hind III -EcoR I DNA fragment of fST103 (6.4 kbp) was mixed with ssDNA of the recombinant phage in 1 x SSC (150 mM NaCl, 15 mM sodium citrate). The mixture was left at 100°C for 7 minutes, transferred into a 65°C bath and allowed to stand for 10 minutes. The gapped duplex DNA-(gd DNA) was purified in a conventinal manner. The gd DNA and mutagenic primers tpA49 and tPA86 (tpA49: 5'-TGGCTCGCTGCAACTGTCGACAGGCACTGAGTGGCA-3' and tPA86: 5'-GCACGTGGCCCTGGTGTCGACTTCACAGCACTTCCC-3') were annealed in the hybridization buffer [150 mM KCl, 50 mM Tris-HCl (pH 7.5)] at 65°C for 7 minutes and then cooled at room temperature for 10 minutes. To the resultant mixture was added filled-in buffer(62.5 mM KCl, 27.5 mM Tris-HCl(pH 7.5), 15 mM MgCl₂, 2 mM DTT, 0.05 mM ATP, and 0.025 mM of each of dATP, dCTP, dGTP and TTP), the mixture was treated with DNA polymerase I Klenow fragment and T4 DNA ligase at room temperature for 45 minutes. E. coli JM103 was transformed with the DNA mixture. The resulting phages were named M13(tPA86-Sal) and M13(tpA49, 86-Sal)and RF DNA of these phages were identified by restriction enzyme analysis. M13(tPA86-Sal) RF DNA and pST118 were digested with Hind III and Bbe I . The resultant fragments (0.48 kbp, 4.8 kbp, respectively) were purified in a conventional manner. These fragment were ligated with T4 DNA ligase and then E. coli MM294 was transformed with the ligation mixture. From one of the ampicilin resistance transformants, the desired plasmid pST118-SS1 was isolated and was identified by restriction enzyme analysis.

The plasmid pST118-SS2 was constructed form M13(tPA49, 86Sal) and pST118 in a similar manner as mentioned above. The resulting plasmid pST118-SS2 was identified by restriction enzyme analysis. The plasmid pST118-SS3 was constructed from pST118-SS2 by deleting the Sal I -Sal I fragment of it. The plasmid pST118-SS2 was digested with Sal I and the large fragment (5.2 kbp) was purified. This fragment was self-ligated with T4 DNA ligase and then E. coli MM294 was transformed with the ligation mixture. From one of the ampicilin resistance transformants, the desired plasmid pST118-SS3 was isolated and identified by restriction enzyme analysis.

## Example 7

### Construction of pplgTQK2

The DNA fragment containing the plasminogen N-terminal peptide gene (2.1 kbp) was removed from the plasmid pplgN1 by digesting it with Hind III, Sca I and Sal I. The DNA fragemnt containing t-PA gene, SV40 polyadenylation site-splicing junction and plasmid pML2 (4.9 Kbp) was removed from the plasmid pST118-SS1 by digesting it with Hind III and Sal I. These fragments were lifted with T4 DNA ligase and then E. coli MM294 was transformed with the ligation mixture. From one of the ampicilin resistant transformants, the desired plasmid pplgTQK1 was isolated and identified by restriction enzyme analysis. To express in mammalian cells, the plasminogen-t-PA hybrid gene was replaced with the native t-PA gene in the plasmid pST112 . Namely, the plasmid pplgTQK2 was constructed by replacing the Bgl II -Sma I fragment (1.5 kbp) in the plasmid pST112 with the Bgl II -Sma I fragment (1.5 kbp) in the plasmid pplgTQK1 in a conventional manner. The resulting plasmid pplgTQK2 was identified by restriction enzyme analysis.

## Example 8

### Construction of plasmid pplgΔGF3

The plasmid pplgN1 was digested with Sal I and then the resulting liner fragment was dephosphorylated by calf intestinal alkaline phosphatase. The dephosphorylated fragment and synthetic oligonucleotide (SBS linker: 5'-TCGAGGGATCCC-3') was joined with T4 DNA ligase and then E. coli MM294 was transformed with the ligation mixture. The resulting plasmid pplgΔGF1 was identified by restriction enzyme analysis. The DNA fragment containing plasminogen N-terminal peptide gene (about 2.2 kbp) was removed from pplgΔGF1 by digesting it with Hind III and BamH I . The DNA fragment comprising t-PA and others (4.9 kbp) was removed from pST118-SS3 by digesting it with Hind III and Bgl II. These Hind III -BamH I fragment and Hind III -Bgl II fragment were joined with T4 DNA ligase and then E. coli MM294 was transfomed with the ligation mixture. From one of the ampicilin resistance transformants, the desired plasmid pplgΔGF2 was isolated and identified by restriction enzyme analysis. To express in mammalian cells, the expression plasmid pplgΔGF3 was constructed by replacing the Bgl II -Sma I fragment (1.5 kbp) in the plasmid pST112 with the Bgl II -Sma I fragment (1.5 kbp) in the plasmid pplgΔGF2 in a conventional manner. The resultant plasmid pplgΔGF3 was identified by restriction enzyme analysis.

Example 9

Transfection with constructed plasmids

Mouse cell lines L-929 was transfected with the above expression plasmids, pplgTQK2 or pplgΔGF3 and marker plasmid pSV2neo encoding G418 resistence gene. After transfection, the resulting G418 resistant colonies were incubated in sterile induction medium [DMEM supplemented with 2% FCS, 1 mM sodium butylate, 60 μM ZnSO₄,15 klU/ml aprotinin] at 37°C for 1 day and the level of t-PA activity and t-PA antigen were determined. Namely, the L-929 cells transfected by the plasmid pplgTQK2 produced the protein pgNTQTPA and the mammalian cells transfected by the plasmid pplgΔGF3 produced the protein plgNΔGFTPA.

Example 10

Expression of plasminogen-t-PA hybrid proteins

The 1.5 x10⁵ the L-929 cells containing plasmid pplgTQK2 cells were seeded at a roller bottle (internal surface area: 680 cm²) in 300 ml of sterile DMEM supplemented with 10% FCS. The cells were incubated at 37°C for 3 days and then the culture medium in the bottle was replaced with 400 ml of the sterile induction medium. After incubation at 37°C for 2 days, the conditioned medium were collected. The yield of plgNTQTPA was measured with ELISA of t-PA [Cf. e.g. Tsutomu Kaizu et al. Thrombs. Res. 40, 91 (1985)] (yield: 330μg/2ℓ of the conditioned medium).

Example 11

Prification of plasminogen-t-PA hybrid proteins

The mutant t-PAs (e.g. plgNTQTPA) were purified with affinity column chromatography.
Anti human t-PA monoclonal antibody [preparation of which is described in e.g.Tsutomu Kaizu et al, Thrombos. Res., 40, 91 (1985)] bounded Sepharose 4B column was equilibrated with the aqueous solution containing 0.1M Tris-HCl(pH 8.0) and 10klU/ml aprotinin. The culture supernatant was applied to the column and then the column was washed with the equilibrated solution. The mutant t-PAs were eluted with aqueous solution containing 0.5 M NaCl, 0.01% Tween 80, 0.1 M glycine-HCl(pH 2.5) and 10 klU/ml Aprotinin. The eluate was rapidly adjusted to pH 8.0 with 1M Tris-HCl(pH 9.0). The neutralized fraction was applied to the p-aminobenzamidine bounded Sepharose 4B column which was equilibrated with aqueous solution containing 1 M NaCl, 0.05% Tween 80, 0.05 M Tris-HCl (pH 8.0) and 10 klU/ml aprotinin in advance. The column was washed with the equilibrated solution and then the mutant t-PAs were eluted with aqueous solution containing 1 M arginine, 0.05% Tween 80, 0.05 M Tris-HCl (pH 8.0) and 10 klU/ml Aprotinin. The eluate was dialyzed three times with the aqueous solution containin g 0.15 M NaCl, 0.05% Tween 80 and 0.1M NH₄HCO₃.The mutant t-PA plgNTQTPA was purified form 2 ℓ of the culture supernatant by the above method (yield 182.9 μg/4.8 ml).
The purified plgNTQTPA was further purified by preparative HPLC using COSMOSIL 5C₄-300(4.6 mmφ x 50 Nacalai Tesque), and sequenced by a gas-phase sequencer 470A (Applied Biosystems Inc). The N-terminal sequence of the sample was Ser-Glu-Pro-Leu-Asp,which was identical with the sequence as expected.

The molecular weight of the purified plgNTQTPA was determined by SDS-PAGE analysis using maker proteins(94,000, 67,000, 45,000, 30,000, 14,400 daltons). The results are shown that the molecular weight is approximately 64,000 dalton(under reduced condition) and 70,500 dalton(under non-reduced condition). It is inferred from the results that thus produced plgNTQTPA is glycosylated, since the found molecular weight is larger than the calculated molecular weight from the sum of the weights of constitutional amino acids of plgNTQTPA.

Example 12

Characterization of mutant t-PA

A. Specific Activity of mutant t-PA

The plasminogen activator activities of mutant t-PAs were determined by indirect spectrophotomeric assay [Cf. Thrombosis Research 31, 427 (1983)]. Namely, various amounts of each mutant t-PA were added to a mixture of 0.2 $\mu$M human plasminogen(The Green Cross Co., Osaka) ,0.9 mM H-D-Val-Leu-Lys-p-nitroanilide$\cdot$2HCl (S-2251, BACHEM) and 70 $\mu$g/ml solubilized fibrin which was prepared by the method of Wiman et.al. [Cf. Clin. Chim. Acta. 127, 279 (1983)] in 50 mM Tris-HCl (pH 8.8) containing 0.1M sodium chloride and 0.01%(v/v) Triton X-100. The reaction mixture was incubated at 37 $^\circ$C for 3 hours. The absorbance at 405 nm was measured against blanks without activator by using a microplate reader(Titertek Multiskan, Flow Laboratories, Inc. USA). Plasminogen activator activity [ International Unit(IU) of t-PA ] of mutant t-PA was determined referring to standard curve of native t-PA [ International standard (WHO) ]. Protein concentration of mutant t-PA was determined by Lowry's method. The results are shown in the following table.

| t-PA | Specific activity (IU/mg) |
|---|---|
| plgNTQTPA | $5.8 \times 10^5$ |
| plg$\Delta$GFTPA | $4.1 \times 10^5$ |

B.In vivo experiment on half life of mutant t-PA

a) Materials:

Animals: All experiments were carried out using male Sprague-Dawley rats(220-245g)anesthetized with Nembutal(50 mg/kg,i.p.).

t-PAs: Native t-PA (single chain, >80%) was purified from the culture medium of human melanoma (Bowes) cells in a conventional manner(e.g. that of Example 11). Recombinant mutant t-PA(plgNTQTPA) was obtained in Example 11.

Other materials: Aprotinin(Boehringer Mannheim GmbH), heparin(Wako pure chemicals,Japan).

b) Method:

A cannula filled with saline was inserted into the femoral vein of the test animal for the injection of t-PA (100 $\mu$g/kg). The left carotid artery of the test animal was cannulated with a polyethylene tube which was filled with heparin(100 units/ml), and at 1,2,3,5,7,10,15 and 20 minutes before and after injection of t-PA, blood samples(200 $\mu$l) were collected in polyethylene tube containing 20 $\mu$l of sodium citrate(3.2%,w/v) and 50 $\mu$l of aprotinin (6000KIU/ml). The blood samples were centrifuged at 10,000 rpm for 2 minutes and the plasmas were recovered. She level of t-PA in the plasma was measured using ELISA for t-PA [Cf. e.g. Tsutomu Kaizu et al., Thrombos. Res. 40, 91 (1985)].

c) Results:

The results are shown in the following table.

| t-PA | half life of t-PA(minutes) |
|---|---|
| Native t-PA | 5.02 |
| plgNTQTPA | >25 |

The expression plasmid, pplgTQK2 or pplgΔGF3 obtained in the above Examples was inserted into the Escherichia coli HB101 and the resultant transformants as mentioned below have been deposited with one of the INTERNATIONAL DEPOSITORY AUTHORITY ON THE BUDAPEST TREATY, Fermentation Research Institute (FRI), Agency of Industrial Science and Technology at 1 -3, Higashi 1 chome, Tsukuba-shi, Ibaraki-ken 305, Japan on January 19, 1989.

| Transformants | Deposit number |
|---|---|
| Escherichia coli HB101(pplgTQK2) | FERM BP-2247 |
| Escherichia coli HB101(pplgΔGF3) | FERM BP-2248 |

New tissue plasminogen activator which has a strong activity for converting plasminogen into plasmin and is useful as a thrombolytic agent comprising a
N-terminal peptide of plasminogen linked to t-PA having a special amino acid sequence;
a DNA encoding amino acid sequence of the t-PA as mentioned;
an expression vector comprising the said DNA and a pharmaceutical composition comprising the said t-PA;
the said t-PA is produced by culturing a host cell transformed with an expression vector comprising DNA encoding amino acid sequence of the said t-PA in a nutrient medium, and recovering the resultant t-PA from the cultured broth.

## Claims

1. N-terminal peptide of plasminogen linked to t-PA.
2. The t-PA of claim 1 having the amino acid sequence:
A$^1$ - A$^2$ - A$^3$     (I)
wherein A$^1$ is
SerGluProLeuAspAspTyrValAsnThrGlnGlyAlaSerLeuPheSerValThrLys                     LysGln-
LeuGlyAlaGlySerIleGluGluCysAlaAlaLysCysGluGluAspGluGlu PheThrCysArgAlaPheGlnTyrHisSerLysGluGln-
GluCysValIleMetAlaGlu AsnArgLysSerSerIleIleArgMetArgAspValValLeuPheGluLysLysVal-,
SerGluProLeuAspAspTyrValAsnThrGlnGlyAlaSerLeuPheSerValThrLys                     LysGln-
LeuGlyAlaGlySerIleGluGluCysAlaAlaLysCysGluGluAspGluGlu PheThrCysArgAlaPheGlnTyrHisSerLysGluGln-
GluCysValIleMetAlaGlu AsnArgLysSerSerIleIleArgMetArgAspValValLeuPheGluLysLysValGlu Gly-,
SerGluProLeuAspAspTyrValAsnThrGlnGlyAlaSerLeuPheSerValThrLys                     LysGln-
LeuGlyAlaGlySerIleGluGluCysAlaAlaLysCysGluGluAspGluGlu         PheThrCysArgAlaPheGlnTyrHisSerLys-
GluGlnGlnCysValIleMetAlaGlu AsnArgLysSerSerIleIleIleArgMetArgAspValValLeuPheGluLysLysVal-or
SerGluProLeuAspAspTyrValAsnThrGlnGlyAlaSerLeuPheSerValThrLys                     LysGln-
LeuGlyAlaGlySerIleGluGluCysAlaAlaLysCysGluGluAspGluGlu         PheThrCysArgAlaPheGlnTyrHisSerLys-

GluGlnGlnCysVallleMetAlaGlu AsnArgLysSerSerIlelleIleArgMetArgAspValValLeuPheGluLysLysVal GluGly-,
A$^1$ is
a bond or the same amino acid sequence as Ser$^1$ to Val$^{48}$ of the native t-PA , and
A$^3$ is
the same amino acid sequence as Asp$^{87}$ to Pro$^{527}$ of the native t-PA.

3. The t-PA of claim 2, in which
A$^1$is
SerGluProLeuAspAspTyrValAsnThrGlnGlyAlaSerLeuPheSerValThrLys                    LysGln-
LeuGlyAlaGlySerIleGluGluCysAlaAlaLysCysGluGluAspGluGlu PheThrCysArgAlaPheGlnTyrHisSerLysGluGln-
GluCysVallleMetAlaGlu AsnArgLysSerSerIlelleArgMetArgAspValValLeuPheGluLysLysValGlu Gly-,
A$^2$ is
the same amino acid sequence as Ser$^1$ to Val$^{48}$ of the native t-PA, and
A$^3$ is
the same amino acid sequence as Asp$^{87}$ to Pro$^{527}$ of the native t-PA.

4. The t-PA of claim 2, in which
A$^1$ is
SerGluProLeuAspAspTyrValAsnThrGlnGlyAlaSerLeuPheSerValThrLys                    LysGln-
LeuGlyAlaGlySerIleGluGluCysAlaAlaLysCysGluGluAspGluGlu PheThrCysArgAlaPheGlnTyrHisSerLysGluGln-
GluCysVallleMetAlaGlu AsnArgLysSerSerIlelleArgMetArgAspValValLeuPheGluLysLysVal-,
A$^2$ is
a bond, and
A$^3$ is
the same amino acid sequence:Asp$^{87}$ to Pro$^{527}$ of the native t-PA.

5. A DNA encoding amino acid sequence of the t-PA as defined in claim 1.
6. A DNA encoding amino acid sequence of the t-PA as defined in claim 2.
7. A expression vector comprising the DNA as defined in claim 5.
8. A expression vector comprising the DNA as defined in claim 6.
9. A host cell transformed with an expression vector as defined in claim 7.
10. A host cell transformed with an expression vector as defined in claim 8.
11. A process for the production of t-PA of claim 1 which comprises, culturing a host cell transfomed with an expression vector comprising DNA encoding t-PA of claim 1 in a nutrient medium, and recovering the resultant t-PA from the cultured broth.
12. A process for the production of t-PA of claim 2 which comprises, culturing a host cell transfomed with an expression vector comprising DNA encoding amino acid sequence of t-PA of claim 2 in a nutrient medium, and recovering the resultant t-PA from the cultured broth.
13. A phamaceutical composition comprising t-PA of claim 1 and pharmaceutically acceptable carrier.

15

Fig.1-(1)
Construction of expression vectors of the new t-PA

Fig. 1-(2)

Fig. 1-(3)

Fig. 2. Restriction Site and Function Map of
Plasmid pMI200

(2.8 Kb)

Coding chain:   5'— GGG···GGTTAAGGGACGCTGTGAAGCAATCATGGATGCA
Coded amino acid:                                                    met asp ala
                                                                      -35

          ATGAAGAGAGGGCTCTGCTGTGTGCTGCTGCTGTGTGGA
          met lys  arg  gly  leu  cys  cys  val  leu  leu  leu  cys  gly

          GCA GTCTTCGTTTCGCCCAGCCAGGAAATCCATGCCCGA
          ala  val  phe  val  ser  pro  ser  gln  glu  ile  his  ala  arg

          TTCAGAAGAGGAGCCAGATCTTACCAAGTGATCCC···CCC
          phe  arg  arg  gly  ala  arg  ser  tyr  gln  val  leu
                                          ↑ 1


Fig.3. DNA sequence of the cDNA insert of plasmid pMI200

EP 0 379 890 A1

Fig.4-(1)DNA sequence of Bgl II DNA fragment(1974bp)

(Upper: Coding chain, Lower: Coded amino acid sequence)

(Bgl.II)
5'-GATCTTACCAAGTGATCTGCAGAGATGAAAAAACGCAGATGATATACCAG
SerTyrGlnValIleCysArgAspGluLysThrGlnMetIleTyrGln
⊢→ Mutant t-PA (Lys277→ Ile)      10

CAACATCAGTCATGGCTGCGCCCTGTGCTCAGAAGCAACCGGGTGGAATATTGCTGGTGC
GlnHisGlnSerTrpLeuArgProValLeuArgSerAsnArgValGluTyrCysTrpCys
·20                                    30

AACAGTGGCAGGGCACAGTGCCACTCAGTGCCTGTCAAAAGTTGCAGCGAGCCAAGGTGT
AsnSerGlyArgAlaGlnCysHisSerValProValLysSerCysSerGluProArgCys
·40                                    50

· TTCAACGGGGGCACCTGCCAGCAGGCCCTGTACTTCTCAGATTTCGTGTGCCAGTGCCCC
PheAsnGlyGlyThrCysGlnGlnAlaLeuTyrPheSerAspPheValCysGlnCysPro
60                                    70
                                              (AvaII)
GAAGGATTTGCTGGGAAGTGCTGTGAAATAGATACCAGGGCCACGTGCTACGAGGACCAG·
·GluGlyPheAlaGlyLysCysCysGluIleAspThrArgAlaThrCysTyrGluAspGln
·80                                    90
                                              (BbeI)
·GGCATCAGCTACAGGGGCACGTGGAGCACAGCGGAGAGTGGCGCCGAGTGCACCAACTGG
·GlyIleSerTyrArgGlyThrTrpSerThrAlaGluSerGlyAlaGluCysThrAsnTrp
-100·                                    ·110

AACAGCAGCGCGTTGGCCCAGAAGCCCTACAGCGGGCGGAGGCCAGACGCCATCAGGCTG
AsnSerSerAlaLeuAlaGlnLysProTyrSerGlyArgArgProAspAlaIleArgLeu
120                                    130

GGCCTGGGGAACCACAACTACTGCAGAAACCCAGATCGAGACTCAAAGCCCTGGTGCTAC
GlyLeuGlyAsnHisAsnTyrCysArgAsnProAspArgAspSerLysProTrpCysTyr
140                                    150
                                              (DdeI)
GTCTTTAAGGCGGGGAAGTACAGCTCAGAGTTCTGCAGCACCCCTGCCTGCTCTGAGGGA
ValPheLysAlaGlyLysTyrSerSerGluPheCysSerThrProAlaCysSerGluGly
160                                    170

AACAGTGACTGCTACTTTGGGAATGGGTCAGCCTACCGTGGCACGCACAGCCTCACCGAG
AsnSerAspCysTyrPheGlyAsnGlySerAlaTyrArgGlyThrHisSerLeuThrGlu
180                                    190
                          (EcoRI)
TCGGGTGCCTCCTGCCTCCCGTGGAATTCCATGATCCTGATAGGCAAGGTTTACACAGCA·
SerGlyAlaSerCysLeuProTrpAsnSerMetIleLeuIleGlyLysValTyrThrAla
200                                    210·

CAGAACCCCAGTGCCCAGGCACTGGGCCTGGGCAAACATAATTACTGCCGGAATCCTGAT
GlnAsnProSerAlaGlnAlaLeuGlyLeuGlyLysHisAsnTyrCysArgAsnProAsp
220·                                    230

Fig. 4-(2)

GGGGATGCCAAGCCCTGGTGCCACGTGCTGAAGAACCGCAGGCTGACGTGGGAGTACTGT
GlyAspAlaLysProTrpCysHisValLeuLysAsnArgArgLeuThrTrpGluTyrCys
240                                    250

GATGTGCCCTCCTGCTCCACCTGCGGCCTGAGACAGTACAGCCAGCCTCAGTTTCGCATC
AspValProSerCysSerThrCysGlyLeuArgGlnTyrSerGlnProGlnPheArgIle
260                                    270

ATAGGAGGCCTCTTCGCCGACATCGCCTCCCACCCCTGGCAGGCTGCCATCTTTGCCAAG
IleGlyGlyLeuPheAlaAspIleAlaSerHisProTrpGlnAlaAlaIlePheAlaLys
280                                    290

CACAGGAGGTCGCCCGGAGAGCGGTTCCTGTGCGGGGGCATACTCATCAGCTCCTGCTGG
HisArgArgSerProGlyGluArgPheLeuCysGlyGlyIleLeuIleSerSerCysTrp
300                                    310

ATTCTCTCTGCCGCCCACTGCTTCCAGGAGAGGTTTCCGCCCCACCACCTGACGGTGATC
IleLeuSerAlaAlaHisCysPheGlnGluArgPheProProHisHisLeuThrValIle
320                                    330

TTGGGCAGAACATACCGGGTGGTCCCTGGCGAGGAGGAGCAGAAATTTGAAGTCGAAAAA
LeuGlyArgThrTyrArgValValProGlyGluGluGluGlnLysPheGluValGluLys
340                                    350

(EcoRI)
TACATTGTCCATAAGGAATTCGATGATGACACTTACGACAATGACATTGCGCTGCTGCAG
TyrIleValHisLysGluPheAspAspAspThrTyrAspAsnAspIleAlaLeuLeuGln
360                                    370

CTGAAATCGGATTCGTCCCGCTGTGCCCAGGAGAGCAGCGTGGTCCGCACTGTGTGCCTT
LeuLysSerAspSerSerArgCysAlaGlnGluSerSerValValArgThrValCysLeu
380                                    390

(SacI)
CCCCCGGCGGACCTGCAGCTGCCGGACTGGACGGAGTGTGAGCTCTCCGGCTACGGCAAG
ProProAlaAspLeuGlnLeuProAspTrpThrGluCysGluLeuSerGlyTyrGlyLys
400                                    410

CATGAGGCCTTGTCTCCTTTCTATTCGGAGCGGCTGAAGGAGGCTCATGTCAGACTGTAC
HisGluAlaLeuSerProPheTyrSerGluArgLeuLysGluAlaHisValArgLeuTyr
420                                    430

CCATCCAGCCGCTGCACATCACAACATTTACTTAACAGAACAGTCACCGACAACATGCTG
ProSerSerArgCysThrSerGlnHisLeuLeuAsnArgThrValThrAspAsnMetLeu
440                                    450

TGTGCTGGAGACACTCGGAGCGGCGGGCCCCAGGCAAACTTGCACGACGCCTGCCAGGGC
CysAlaGlyAspThrArgSerGlyGlyProGlnAlaAsnLeuHisAspAlaCysGlnGly
460                                    470

GATTCGGGAGGCCCCCTGGTGTGTCTGAACGATGGCCGCATGACTTTGGTGGGCATCATC
AspSerGlyGlyProLeuValCysLeuAsnAspGlyArgMetThrLeuValGlyIleIle
480                                    490

Fig. 4-(3)

```
AGCTGGGGCCTGGGCTGTGGACAGAAGGATGTCCCGGGTGTGTACACAAAGGTTACCAAC
SerTrpGlyLeuGlyCysGlyGlnLysAspValProGlyValTyrThrLysValThrAsn
        500                                       510

TACCTAGACTGGATTCGTGACAACATGCGACCGTGACCAGGAACACCCGACTCCTCAAAA
TyrLeuAspTrpIleArgAspAsnMetArgPro***|——→ Noncoding
        520
```

(Sau3AI)
```
GCAAATGAGATCCCGCCTCTTCTTCTTCAGAAGACACTGCAAAGGCGCAGTGCTTCTCTA
```

```
CAGACTTCTCCAGACCCACCACACCGCAGAAGCGGGACGAGACCCTACAGGAGAGGGAAG
```

```
AGTGCATTTTCCCAGATACTTCCCATTTTGGAAGTTTTCAGGACTTGGTCTGATTTCAGG
```

```
ATACTCTGTCAGATGGGAAGACATGAATGCACACTAGCCTCTCCAGGAATGCCTCCTCCC
```

```
TGGGCAGAAGTGGCCATGCCACCCTGTTTTCGCTAAAGCCCAACCTCCTGACCTGTCACC
```

```
GTGAGCAGCTTTGGAAACAGGACCACAAAAATGAAAGCATGTCTCAATAGTAAAAGAAAC
```

(Bgl II)

AAGA -3'

Fig.5.  Restriction Site and Function Map of
Plasmid pMI205
(4.8 Kb)

Fig. 6   Restriction Site and Function Map of
Plasmid pST112
(7.3 Kb)

Fig. 7-(1). cDNA sequence of a native tPA in pST112
(Upper: Coding chain
Lower: Coded amino acid sequence)

```
           10        20        30        40        50        60
5'- GTTAAGGGACGCTGTGAAGCAATCATGGATGCAATGAAGAGAGGGCTCTGCTGTGTGCTG
                             MetAspAlaMetLysArgGlyLeuCysCysValLeu

           70        80        90       100       110       120
    CTGCTGTGTGGAGCAGTCTTCGTTTCGCCCAGCCAGGAAATCCATGCCCGATTCAGAAGA
    LeuLeuCysGlyAlaValPheValSerProSerGlnGluIleHisAlaArgPheArgArg

          130       140       150       160       170       180
    GGAGCCAGATCTTACCAAGTGATCTGCAGAGATGAAAAAACGCAGATGATATACCAGCAA
    GlyAlaArgSerTyrGlnValIleCysArgAspGluLysThrGlnMetIleTyrGlnGln

    native tPA
          190       200       210       220       230       240
    CATCAGTCATGGCTGCGCCCTGTGCTCAGAAGCAACCGGGTGGAATATTGCTGGTGCAAC
    HisGlnSerTrpLeuArgProValLeuArgSerAsnArgValGluTyrCysTrpCysAsn

          250       260       270       280       290       300
    AGTGGCAGGGCACAGTGCCACTCAGTGCCTGTCAAAAGTTGCAGCGAGCCAAGGTGTTTC
    SerGlyArgAlaGlnCysHisSerValProValLysSerCysSerGluProArgCysPhe

          310       320       330       340       350       360
    AACGGGGGCACCTGCCAGCAGGCCCTGTACTTCTCAGATTTCGTGTGCCAGTGCCCCGAA
    AsnGlyGlyThrCysGlnGlnAlaLeuTyrPheSerAspPheValCysGlnCysProGlu

          370       380       390       400       410       420
    GGATTTGCTGGGAAGTGCTGTGAAATAGATACCAGGGCCACGTGCTACGAGGACCAGGGC
    GlyPheAlaGlyLysCysCysGluIleAspThrArgAlaThrCysTyrGluAspGlnGly

          430       440       450       460       470       480
    ATCAGCTACAGGGGCACGTGGAGCACAGCGGAGAGTGGCGCCGAGTGCACCAACTGGAAC
    IleSerTyrArgGlyThrTrpSerThrAlaGluSerGlyAlaGluCysThrAsnTrpAsn

          490       500       510       520       530       540
    AGCAGCGCGTTGGCCCCAGAAGCCCTACAGCGGGCGGAGGCCAGACGCCATCAGGCTGGGC
    SerSerAlaLeuAlaGlnLysProTyrSerGlyArgArgProAspAlaIleArgLeuGly
```

Fig. 7-(2).

```
          550          560          570          580          590          600
CTGGGGAACCACAACTACTGCAGAAACCCAGATCGAGACTCAAAGCCCTGGTGCTACGTC
LeuGlyAsnHisAsnTyrCysArgAsnProAspArgAspSerLysProTrpCysTyrVal

          610          620          630          640          650          660
TTTAAGGCGGGGAAGTACAGCTCAGAGTTCTGCAGCACCCCTGCCTGCTCTGAGGGAAAC
PheLysAlaGlyLysTyrSerSerGluPheCysSerThrProAlaCysSerGluGlyAsn

          670          680          690          700          710          720
AGTGACTGCTACTTTGGGAATGGGTCAGCCTACCGTGGCACGCACAGCCTCACCGAGTCG
SerAspCysTyrPheGlyAsnGlySerAlaTyrArgGlyThrHisSerLeuThrGluSer

          730          740          750          760          770          780
GGTGCCTCCTGCCTCCCGTGGAATTCCATGATCCTGATAGGCAAGGTTTACACAGCACAG
GlyAlaSerCysLeuProTrpAsnSerMetIleLeuIleGlyLysValTyrThrAlaGln

          790          800          810          820          830          840
AACCCCAGTGCCCAGGCACTGGGCCTGGGCAAACATAATTACTGCCGGAATCCTGATGGG
AsnProSerAlaGlnAlaLeuGlyLeuGlyLysHisAsnTyrCysArgAsnProAspGly

          850          860          870          880          890          900
GATGCCAAGCCCTGGTGCCACGTGCTGAAGAACCGCAGGCTGACGTGGGAGTACTGTGAT
AspAlaLysProTrpCysHisValLeuLysAsnArgArgLeuThrTrpGluTyrCysAsp

          910          920          930          940          950          960
GTGCCCTCCTGCTCCACCTGCGGCCTGAGACAGTACAGCCAGCCTCAGTTTCGCATCAAA
ValProSerCysSerThrCysGlyLeuArgGlnTyrSerGlnProGlnPheArgIleLys

          970          980          990         1000         1010         1020
GGAGGGCTCTTCGCCGACATCGCCTCCCACCCCTGGCAGGCTGCCATCTTTGCCAAGCAC
GlyGlyLeuPheAlaAspIleAlaSerHisProTrpGlnAlaAlaIlePheAlaLysHis

         1030         1040         1050         1060         1070         1080
AGGAGGTCGCCCGGAGAGCGGTTCCTGTGCGGGGGCATACTCATCAGCTCCTGCTGGATT
ArgArgSerProGlyGluArgPheLeuCysGlyGlyIleLeuIleSerSerCysTrpIle
```

Fig. 7-(3).

```
          1090       1100       1110       1120       1130       1140
CTCTCTGCCGCCCACTGCTTCCAGGAGAGGTTTCCGCCCCACCACCTGACGGTGATCTTG
LeuSerAlaAlaHisCysPheGlnGluArgPheProProHisHisLeuThrValIleLeu


          1150       1160       1170       1180       1190       1200
GGCAGAACATACCGGGTGGTCCCTGGCGAGGAGGAGCAGAAATTTGAAGTCGAAAAATAC
GlyArgThrTyrArgValValProGlyGluGluGluGlnLysPheGluValGluLysTyr


          1210       1220       1230       1240       1250       1260
ATTGTCCATAAGGAATTCGATGATGACACTTACGACAATGACATTGCGCTGCTGCAGCTG
IleValHisLysGluPheAspAspAspThrTyrAspAsnAspIleAlaLeuLeuGlnLeu


          1270       1280       1290       1300       1310       1320
AAATCGGATTCGTCCCGCTGTGCCCAGGAGAGCAGCGTGGTCCGCACTGTGTGCCTTCCC
LysSerAspSerSerArgCysAlaGlnGluSerSerValValArgThrValCysLeuPro


          1330       1340       1350       1360       1370       1380
CCGGCGGACCTGCAGCTGCCGGACTGGACGGAGTGTGAGCTCTCCGGCTACGGCAAGCAT
ProAlaAspLeuGlnLeuProAspTrpThrGluCysGluLeuSerGlyTyrGlyLysHis


          1390       1400       1410       1420       1430       1440
GAGGCCTTGTCTCCTTTCTATTCGGAGCGGCTGAAGGAGGCTCATGTCAGACTGTACCCA
GluAlaLeuSerProPheTyrSerGluArgLeuLysGluAlaHisValArgLeuTyrPro


          1450       1460       1470       1480       1490       1500
TCCAGCCGCTGCACATCACAACATTTACTTAACAGAACAGTCACCGACAACATGCTGTGT
SerSerArgCysThrSerGlnHisLeuLeuAsnArgThrValThrAspAsnMetLeuCys


          1510       1520       1530       1540       1550       1560
GCTGGAGACACTCGGAGCGGCGGGCCCCAGGCAAACTTGCACGACGCCTGCCAGGGCGAT
AlaGlyAspThrArgSerGlyGlyProGlnAlaAsnLeuHisAspAlaCysGlnGlyAsp
```

Fig. 7-(4).

```
          1570        1580        1590        1600        1610        1620
TCGGGAGGCCCCCTGGTGTGTCTGAACGATGGCCGCATGACTTTGGTGGGCATCATCAGC
SerGlyGlyProLeuValCysLeuAsnAspGlyArgMetThrLeuValGlyIleIleSer

          1630        1640        1650        1660        1670        1680
TGGGGCCTGGGCTGTGGACAGAAGGATGTCCCGGGTGTGTACACAAAGGTTACCAACTAC
TrpGlyLeuGlyCysGlyGlnLysAspValProGlyValTyrThrLysValThrAsnTyr

          1690        1700        1710        1720        1730        1740
CTAGACTGGATTCGTGACAACATGCGACCGTGACCAGGAACACCCGACTCCTCAAAAGCA
LeuAspTrpIleArgAspAsnMetArgPro***

          1750        1760        1770        1780        1790        1800
AATGAGATCCCGCCTCTTCTTCTTCAGAAGACACTGCAAAGGCGCAGTGCTTCTCTACAG

          1810        1820        1830        1840        1850        1860
ACTTCTCCAGACCCACCACACCGCAGAAGCGGGACGAGACCCTACAGGAGAGGGAAGAGT

          1870        1880        1890        1900        1910        1920
GCATTTTCCCAGATACTTCCCATTTTGGAAGTTTTCAGGACTTGGTCTGATTTCAGGATA

          1930        1940        1950        1960        1970        1980
CTCTGTCAGATGGGAAGACATGAATGCACACTAGCCTCTCCAGGAATGCCTCCTCCCTGG

          1990        2000        2010        2020        2030        2040
GCAGAAGTGGCCATGCCACCCTGTTTTCGCTAAAGCCCAACCTCCTGACCTGTCACCGTG

          2050        2060        2070        2080        2090        2100
AGCAGCTTTGGAAACAGGACCACAAAAATGAAAGCATGTCTCAATAGTAAAAGAAACAAG
```

A −3'

Fig.8.    Restriction Site and Function Map of
Plasmid pST118
(7.0 Kb)

Fig. 9. Construction of plasminogen N-terminal peptide gene


plg N1:5'-AGATCTGAGC CCCTCGACGA TTACGTGAAC ACCCAAGGCG CCAGCCTGTT
(78mer)
        CTCCGTGACC AAGAAACAGC TGGGTGCT-3'


plg N2:5'-GGCTCTATCG AAGAGTGCGC CGCTAAGTGT GAAGAGGACG AGGAATTTAC
(80mer)
        TTGCAGGGCC TTCCAGTACC ACAGCAAGGA-3'


plg N3:5'-ACAGGAGTGT GTGATCATGG CCGAGAACCG CAAATCCTCT ATCATTAGAA
(79mer)
        TGAGGGACGT CGTGCTGTTC GAGAAGAAG-3'


plg N4:5'-GTCGACCTTC TTCTCGAACA GCACGACGTC CCTCATTCTA ATGATAGAGG
(74mer)
        ATTTGCGGTT CTCGGCCATG ATCA-3'


plg N5:5'-CACACTCCTG TTCCTTGCTG TGGTACTGGA AGGCCCTGCA AGTAAATTCC
(81mer)
        TCGTCCTCTT CACACTTAGC GGCGCACTCT T-3'


plg N6:5'-CGATAGAGCC GACACCCAGC TGTTTCTTGG TCACGGAGAA CAGGCTGGCG
(82mer)
        CCTTGGGTGT TCACGTAATC GTCGAGGGGC TC-3'

| plgN1 | plgN2 | plgN3 |
|-------|-------|-------|
| plgN6 | plgN5 | plgN4 |

— phosphorylation and ligation
— digestion with Bgl II and Sal I

```
         10          20          30          40          50          60
5'-GATCTGAGCC CCTCGACGAT TACGTGAACA CCCAAGGCGC CAGCCTGTTC TCCGTGACCA
3'-ACTCGG GGAGCTGCTA ATGCACTTGT GGGTTCCGCG GTCGGACAAG AGGCACTGGT

         70          80          90         100         110         120
   AGAAACAGCT GGGTGCTGGC TCTATCGAAG AGTGCGCCGC TAAGTGTGAA GAGGACGAGG
   TCTTTGTCGA CCCACGACCG AGATAGCTTC TCACGCGGCG ATTCACACTT CTCCTGCTCC

        130         140         150         160         170         180
   AATTTACTTG CAGGGCCTTC CAGTACCACA GCAAGGAACA GGAGTGTGTG ATCATGGCCG
   TTAAATGAAC GTCCCGGAAG GTCATGGTGT CGTTCCTTGT CCTCACACAC TAGTACCGGC

        190         200         210         220         230         240
   AGAACCGCAA ATCCTCTATC ATTAGAATGA GGGACGTCGT GCTGTTCGAG AAGAAGG-3'
   TCTTGGCGTT TAGGAGATAG TAATCTTACT CCCTGCAGCA CGACAAGCTC TTCTTCCAGCT-5
```

Fig.10. Restriction Site and Function Map of
Plasmid pplgN1
(4.4 Kb)

Fig.11. Restriction site and Function Map of
Plasmid fST103
(7.1 Kb)

Fig.12 . Restriction Site and Function Map of
Plasmid M13(tPA86-Sal)   (7.1 Kb)

Fig.13.  Restriction Site and Function Map of
Plasmid M13(tPA49,86-Sal)  (7.1 Kb)

Fig.14.  Restriction Site and Function Map of
Plasmid pST118-SS1  (5.3 Kb)

Fig. 15. Restriction Site and Function Map of
Plasmid pST118-SS2 (5.3 Kb)

Fig. 16. Restriction Site and Function Map of Plasmid pST118-SS3 (5. 2 Kb)

Fig.17.   Restriction Site and Function Map of
Plasmid pplgTQK1   (7.0 Kb)

Fig.18. Restriction Site and Function Map of
Plasmid pplgIQK2 (7.3 Kb)

Fig. 19. Restriction Site and Function Map of
Plasmid pplg△GF1
(4.4 Kb)

Fig. 20. Restriction Site and Function Map of
Plasmid pplg△GF2 (7.1 Kb)

Fig.21.  Restriction Site and Function Map of
Plasmid pplg△GF3  (7.4 Kb)

Fig. 22-(1). DNA sequence of mutated t-PA cDNA encoding plgNTQTPA as mentioned in Figure 18 and the corresponding amino acid sequence

(Upper: Coding chain
Lower: Coded amino acid sequence)

```
                    5'-AAGCTTGGCTGCAGGGGGGGTTAAGGGACGCTGTGAAGCAATC

         10        20        30        40        50        60
ATGGATGCAATGAAGAGAGGGCTCTGCTGTGTGCTGCTGCTGTGTGGAGCAGTCTTCGTT
MetAspAlaMetLysArgGlyLeuCysCysValLeuLeuLeuCysGlyAlaValPheVal

         70        80        90       100       110       120
TCGCCCAGCCAGGAAATCCATGCCCGATTCAGAAGAGGAGCCAGATCTGAGCCCCTCGAC
SerProSerGlnGluIleHisAlaArgPheArgArgGlyAlaArgSerGluProLeuAsp
                                                  |——————→plgNTQTPA
        130       140       150       160       170       180
GATTACGTGAACACCCAAGGCGCCAGCCTGTTCTCCGTGACCAAGAAACAGCTGGGTGCT
AspTyrValAsnThrGlnGlyAlaSerLeuPheSerValThrLysLysGlnLeuGlyAla

        190       200       210       220       230       240
GGCTCTATCGAAGAGTGCGCCGCTAAGTGTGAAGAGGACGAGGAATTTACTTGCAGGGCC
GlySerIleGluGluCysAlaAlaLysCysGluGluAspGluGluPheThrCysArgAla

        250       260       270       280       290       300
TTCCAGTACCACAGCAAGGAACAGGAGTGTGTGATCATGGCCGAGAACCGCAAATCCTCT
PheGlnTyrHisSerLysGluGlnGluCysValIleMetAlaGluAsnArgLysSerSer

        310       320       330       340       350       360
ATCATTAGAATGAGGGACGTCGTGCTGTTCGAGAAGAAGGTCGACACCAGGGCCACGTGC
IleIleArgMetArgAspValValLeuPheGluLysLysValAspThrArgAlaThrCys

        370       380       390       400       410       420
TACGAGGACCAGGGCATCAGCTACAGGGGCACGTGGAGCACAGCGGAGAGTGGCGCCGAG
TyrGluAspGlnGlyIleSerTyrArgGlyThrTrpSerThrAlaGluSerGlyAlaGlu

        430       440       450       460       470       480
TGCACCAACTGGAACAGCAGCGCGTTGGCCCAGAAGCCCTACAGCGGGCGGAGGCCAGAC
CysThrAsnTrpAsnSerSerAlaLeuAlaGlnLysProTyrSerGlyArgArgProAsp

        490       500       510       520       530       540
GCCATCAGGCTGGGCCTGGGGAACCACAACTACTGCAGAAACCCAGATCGAGACTCAAAG
AlaIleArgLeuGlyLeuGlyAsnHisAsnTyrCysArgAsnProAspArgAspSerLys

        550       560       570       580       590       600
CCCTGGTGCTACGTCTTTAAGGCGGGGAAGTACAGCTCAGAGTTCTGCAGCACCCCTGCC
ProTrpCysTyrValPheLysAlaGlyLysTyrSerSerGluPheCysSerThrProAla

        610       620       630       640       650       660
TGCTCTGAGGGAAACAGTGACTGCTACTTTGGGAATGGGTCAGCCTACCGTGGCACGCAC
CysSerGluGlyAsnSerAspCysTyrPheGlyAsnGlySerAlaTyrArgGlyThrHis

        670       680       690       700       710       720
AGCCTCACCGAGTCGGGTGCCTCCTGCCTCCCGTGGAATTCCATGATCCTGATAGGCAAG
SerLeuThrGluSerGlyAlaSerCysLeuProTrpAsnSerMetIleLeuIleGlyLys
```

Fig. 22-(2).

```
        730       740       750       760       770       780
GTTTACACAGCACAGAACCCCAGTGCCCAGGCACTGGGCCTGGGCAAACATAATTACTGC
ValTyrThrAlaGlnAsnProSerAlaGlnAlaLeuGlyLeuGlyLysHisAsnTyrCys

        790       800       810       820       830       840
CGGAATCCTGATGGGGATGCCAAGCCCTGGTGCCACGTGCTGAAGAACCGCAGGCTGACG
ArgAsnProAspGlyAspAlaLysProTrpCysHisValLeuLysAsnArgArgLeuThr

        850       860       870       880       890       900
TGGGAGTACTGTGATGTGCCCTCCTGCTCCACCTGCGGCCTGAGACAGTACAGCCAGCCT
TrpGluTyrCysAspValProSerCysSerThrCysGlyLeuArgGlnTyrSerGlnPro

        910       920       930       940       950       960
CAGTTTCGCATCAAAGGAGGGCTCTTCGCCGACATCGCCTCCCACCCCTGGCAGGCTGCC
GlnPheArgIleLysGlyGlyLeuPheAlaAspIleAlaSerHisProTrpGlnAlaAla

        970       980       990      1000      1010      1020
ATCTTTGCCAAGCACAGGAGGTCGCCCGGAGAGCGGTTCCTGTGCGGGGGCATACTCATC
IlePheAlaLysHisArgArgSerProGlyGluArgPheLeuCysGlyGlyIleLeuIle

       1030      1040      1050      1060      1070      1080
AGCTCCTGCTGGATTCTCTCTGCCGCCCACTGCTTCCAGGAGAGGTTTCCGCCCCACCAC
SerSerCysTrpIleLeuSerAlaAlaHisCysPheGlnGluArgPheProProHisHis

       1090      1100      1110      1120      1130      1140
CTGACGGTGATCTTGGGCAGAACATACCGGGTGGTCCCTGGCGAGGAGGAGCAGAAATTT
LeuThrValIleLeuGlyArgThrTyrArgValValProGlyGluGluGluGlnLysPhe

       1150      1160      1170      1180      1190      1200
GAAGTCGAAAAATACATTGTCCATAAGGAATTCGATGATGACACTTACGACAATGACATT
GluValGluLysTyrIleValHisLysGluPheAspAspAspThrTyrAspAsnAspIle

       1210      1220      1230      1240      1250      1260
GCGCTGCTGCAGCTGAAATCGGATTCGTCCCGCTGTGCCCAGGAGAGCAGCGTGGTCCGC
AlaLeuLeuGlnLeuLysSerAspSerSerArgCysAlaGlnGluSerSerValValArg

       1270      1280      1290      1300      1310      1320
ACTGTGTGCCTTCCCCCGGCGGACCTGCAGCTGCCGGACTGGACGGAGTGTGAGCTCTCC
ThrValCysLeuProProAlaAspLeuGlnLeuProAspTrpThrGluCysGluLeuSer

       1330      1340      1350      1360      1370      1380
GGCTACGGCAAGCATGAGGCCTTGTCTCCTTTCTATTCGGAGCGGCTGAAGGAGGCTCAT
GlyTyrGlyLysHisGluAlaLeuSerProPheTyrSerGluArgLeuLysGluAlaHis
```

Fig. 22-(3̄).

```
        1390        1400        1410        1420        1430        1440
GTCAGACTGTACCCATCCAGCCGCTGCACATCACAACATTTACTTAACAGAACAGTCACC
ValArgLeuTyrProSerSerArgCysThrSerGlnHisLeuLeuAsnArgThrValThr

        1450        1460        1470        1480        1490        1500
GACAACATGCTGTGTGCTGGAGACACTCGGAGCGGCGGGCCCCAGGCAAACTTGCACGAC
AspAsnMetLeuCysAlaGlyAspThrArgSerGlyGlyProGlnAlaAsnLeuHisAsp

        1510        1520        1530        1540        1550        1560
GCCTGCCAGGGCGATTCGGGAGGCCCCCTGGTGTGTCTGAACGATGGCCGCATGACTTTG
AlaCysGlnGlyAspSerGlyGlyProLeuValCysLeuAsnAspGlyArgMetThrLeu

        1570        1580        1590        1600        1610        1620
GTGGGCATCATCAGCTGGGGCCTGGGCTGTGGACAGAAGGATGTCCCGGGTGTGTACACA
ValGlyIleIleSerTrpGlyLeuGlyCysGlyGlnLysAspValProGlyValTyrThr

        1630        1640        1650        1660        1670
AAGGTTACCAACTACCTAGACTGGATTCGTGACAACATGCGACCGTGACCAGGAACACCC
LysValThrAsnTyrLeuAspTrpIleArgAspAsnMetArgPro***
                                            ←——|
```

GACTCCTCAAAAGCAAATGAGATCCCGCCTCTTCTTCTTCAGAAGACACTGCAAAGGCGC


AGTGCTTCTCTACAGACTTCTCCAGACCCACCACACCGCAGAAGCGGGACGAGACCCTAC


AGGAGAGGGAAGAGTGCATTTTCCCAGATACTTCCCATTTTGGAAGTTTTCAGGACTTGG


TCTGATTTCAGGATACTCTGTCAGATGGGAAGACATGAATGCACACTAGCCTCTCCAGGA


ATGCCTCCTCCCTGGGCAGAAGTGGCCATGCCACCCTGTTTTCGCTAAAGCCCAACCTCC


TGACCTGTCACCGTGAGCAGCTTTGGAAACAGGACCACAAAAATGAAAGCATGTCTCAAT


AGTAAAAGAAACAAGA  -3'

Fig. 23-(1). DNA sequence of mutated t-PA cDNA encoding plgNΔGFTPA as mentioned in Figure 21 and the corresponding amino acid sequence

(Upper: Coding chain
Lower: Coded amino acid sequence)

5'-AAGCTTGGCTGCAGGGGGGGGTTAAGGGACGCTGTGAAGCAATC

```
              10        20        30        40        50        60
     ATGGATGCAATGAAGAGAGGGCTCTGCTGTGTGCTGCTGCTGTGTGGAGCAGTCTTCGTT
     MetAspAlaMetLysArgGlyLeuCysCysValLeuLeuLeuCysGlyAlaValPheVal

              70        80        90       100       110       120
     TCGCCCAGCCAGGAAATCCATGCCCGATTCAGAAGAGGAGCCAGATCTGAGCCCCTCGAC
     SerProSerGlnGluIleHisAlaArgPheArgArgGlyAlaArgSerGluProLeuAsp
                                                    |——→ plgNΔGFTPA
             130       140       150       160       170       180
     GATTACGTGAACACCCAAGGCGCCAGCCTGTTCTCCGTGACCAAGAAACAGCTGGGTGCT
     AspTyrValAsnThrGlnGlyAlaSerLeuPheSerValThrLysLysGlnLeuGlyAla

             190       200       210       220       230       240
     GGCTCTATCGAAGAGTGCGCCGCTAAGTGTGAAGAGGACGAGGAATTTACTTGCAGGGCC
     GlySerIleGluGluCysAlaAlaLysCysGluGluAspGluGluPheThrCysArgAla

             250       260       270       280       290       300
     TTCCAGTACCACAGCAAGGAACAGGAGTGTGTGATCATGGCCGAGAACCGCAAATCCTCT
     PheGlnTyrHisSerLysGluGlnGluCysValIleMetAlaGluAsnArgLysSerSer

             310       320       330       340       350       360
     ATCATTAGAATGAGGGACGTCGTGCTGTTCGAGAAGAAGGTCGAGGGATCTTACCAAGTG
     IleIleArgMetArgAspValValLeuPheGluLysLysValGluGlySerTyrGlnVal

             370       380       390       400       410       420
     ATCTGCAGAGATGAAAAAACGCAGATGATATACCAGCAACATCAGTCATGGCTGCGCCCT
     IleCysArgAspGluLysThrGlnMetIleTyrGlnGlnHisGlnSerTrpLeuArgPro

             430       440       450       460       470       480
     GTGCTCAGAAGCAACCGGGTGGAATATTGCTGGTGCAACAGTGGCAGGGCACAGTGCCAC
     ValLeuArgSerAsnArgValGluTyrCysTrpCysAsnSerGlyArgAlaGlnCysHis

             490       500       510       520       530       540
     TCAGTGCCTGTCGACACCAGGGCCACGTGCTACGAGGACCAGGGCATCAGCTACAGGGGC
     SerValProValAspThrArgAlaThrCysTyrGluAspGlnGlyIleSerTyrArgGly

             550       560       570       580       590       600
     ACGTGGAGCACAGCGGAGAGTGGCGCCGAGTGCACCAACTGGAACAGCAGCGCGTTGGCC
     ThrTrpSerThrAlaGluSerGlyAlaGluCysThrAsnTrpAsnSerSerAlaLeuAla

             610       620       630       640       650       660
     CAGAAGCCCTACAGCGGGCGGAGGCCAGACGCCATCAGGCTGGGCCTGGGGAACCACAAC
     GlnLysProTyrSerGlyArgArgProAspAlaIleArgLeuGlyLeuGlyAsnHisAsn
```

Fig. 23-(2).

```
        670      680      690      700      710      720
TACTGCAGAAACCCAGATCGAGACTCAAAGCCCTGGTGCTACGTCTTTAAGGCGGGGAAG
TyrCysArgAsnProAspArgAspSerLysProTrpCysTyrValPheLysAlaGlyLys

        730      740      750      760      770      780
TACAGCTCAGAGTTCTGCAGCACCCCTGCCTGCTCTGAGGGAAACAGTGACTGCTACTTT
TyrSerSerGluPheCysSerThrProAlaCysSerGluGlyAsnSerAspCysTyrPhe

        790      800      810      820      830      840
GGGAATGGGTCAGCCTACCGTGGCACGCACAGCCTCACCGAGTCGGGTGCCTCCTGCCTC
GlyAsnGlySerAlaTyrArgGlyThrHisSerLeuThrGluSerGlyAlaSerCysLeu

        850      860      870      880      890      900
CCGTGGAATTCCATGATCCTGATAGGCAAGGTTTACACAGCACAGAACCCCAGTGCCCAG
ProTrpAsnSerMetIleLeuIleGlyLysValTyrThrAlaGlnAsnProSerAlaGln

        910      920      930      940      950      960
GCACTGGGCCTGGGCAAACATAATTACTGCCGGAATCCTGATGGGGATGCCAAGCCCTGG
AlaLeuGlyLeuGlyLysHisAsnTyrCysArgAsnProAspGlyAspAlaLysProTrp

        970      980      990     1000     1010     1020
TGCCACGTGCTGAAGAACCGCAGGCTGACGTGGGAGTACTGTGATGTGCCCTCCTGCTCC
CysHisValLeuLysAsnArgArgLeuThrTrpGluTyrCysAspValProSerCysSer

       1030     1040     1050     1060     1070     1080
ACCTGCGGCCTGAGACAGTACAGCCAGCCTCAGTTTCGCATCAAAGGAGGGCTCTTCGCC
ThrCysGlyLeuArgGlnTyrSerGlnProGlnPheArgIleLysGlyGlyLeuPheAla

       1090     1100     1110     1120     1130     1140
GACATCGCCTCCCACCCCTGGCAGGCTGCCATCTTTGCCAAGCACAGGAGGTCGCCCGGA
AspIleAlaSerHisProTrpGlnAlaAlaIlePheAlaLysHisArgArgSerProGly

       1150     1160     1170     1180     1190     1200
GAGCGGTTCCTGTGCGGGGGGCATACTCATCAGCTCCTGCTGGATTCTCTCTGCCGCCCAC
GluArgPheLeuCysGlyGlyIleLeuIleSerSerCysTrpIleLeuSerAlaAlaHis

       1210     1220     1230     1240     1250     1260
TGCTTCCAGGAGAGGTTTCCGCCCCACCACCTGACGGTGATCTTGGGCAGAACATACCGG
CysPheGlnGluArgPheProProHisHisLeuThrValIleLeuGlyArgThrTyrArg

       1270     1280     1290     1300     1310     1320
GTGGTCCCTGGCGAGGAGGAGCAGAAATTTGAAGTCGAAAAATACATTGTCCATAAGGAA
ValValProGlyGluGluGluGlnLysPheGluValGluLysTyrIleValHisLysGlu

       1330     1340     1350     1360     1370     1380
TTCGATGATGACACTTACGACAATGACATTGCGCTGCTGCAGCTGAAATCGGATTCGTCC
PheAspAspAspThrTyrAspAsnAspIleAlaLeuLeuGlnLeuLysSerAspSerSer

       1390     1400     1410     1420     1430     1440
CGCTGTGCCCAGGAGAGCAGCGTGGTCCGCACTGTGTGCCTTCCCCCGGCGGACCTGCAG
ArgCysAlaGlnGluSerSerValValArgThrValCysLeuProProAlaAspLeuGln
```

Fig. 23-(3).

```
      1450      1460      1470      1480      1490      1500
CTGCCGGACTGGACGGAGTGTGAGCTCTCCGGCTACGGCAAGCATGAGGCCTTGTCTCCT
LeuProAspTrpThrGluCysGluLeuSerGlyTyrGlyLysHisGluAlaLeuSerPro

      1510      1520      1530      1540      1550      1560
TTCTATTCGGAGCGGCTGAAGGAGGCTCATGTCAGACTGTACCCATCCAGCCGCTGCACA
PheTyrSerGluArgLeuLysGluAlaHisValArgLeuTyrProSerSerArgCysThr

      1570      1580      1590      1600      1610      1620
TCACAACATTTACTTAACAGAACAGTCACCGACAACATGCTGTGTGCTGGAGACACTCGG
SerGlnHisLeuLeuAsnArgThrValThrAspAsnMetLeuCysAlaGlyAspThrArg

      1630      1640      1650      1660      1670      1680
AGCGGCGGGCCCCAGGCAAACTTGCACGACGCCTGCCAGGGCGATTCGGGAGGCCCCCTG
SerGlyGlyProGlnAlaAsnLeuHisAspAlaCysGlnGlyAspSerGlyGlyProLeu

      1690      1700      1710      1720      1730      1740
GTGTGTCTGAACGATGGCCGCATGACTTTGGTGGGCATCATCAGCTGGGGCCTGGGCTGT
ValCysLeuAsnAspGlyArgMetThrLeuValGlyIleIleSerTrpGlyLeuGlyCys

      1750      1760      1770      1780      1790      1800
GGACAGAAGGATGTCCCGGGTGTGTACACAAAGGTTACCAACTACCTAGACTGGATTCGT
GlyGlnLysAspValProGlyValTyrThrLysValThrAsnTyrLeuAspTrpIleArg

      1810        1820
GACAACATGCGACCGTGACCAGGAACACCCGACTCCTCAAAAGCAAATGAGATCCCGCCT
AspAsnMetArgPro***
```

CTTCTTCTTCAGAAGACACTGCAAAGGCGCAGTGCTTCTCTACAGACTTCTCCAGACCCA

CCACACCGCAGAAGCGGGACGAGACCCTACAGGAGAGGGAAGAGTGCATTTTCCCAGATA

CTTCCCATTTTGGAAGTTTTCAGGACTTGGTCTGATTTCAGGATACTCTGTCAGATGGGA

AGACATGAATGCACACTAGCCTCTCCAGGAATGCCTCCTCCCTGGGCAGAAGTGGCCATG

CCACCCTGTTTTCGCTAAAGCCCAACCTCCTGACCTGTCACCGTGAGCAGCTTTGGAAAC

AGGACCACAAAAAATGAAAGCATGTCTCAATAGTAAAAGAAACAAGA -3'

| European Patent Office | EUROPEAN SEARCH REPORT | Application Number |
|---|---|---|

EP 90 10 0457

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 292 326 (BEECHAM GROUP PLC) * Claims * | 1,5,7,9 ,11-13 | C 12 N 15/62 |
| | --- | | C 12 N 15/58 |
| A | BIOCHEMISTRY, vol. 26, 1987, pages 4661-4667, American Chemical Society, Washington DC, US; K.C. ROBBINS et al.: "A covalent molecular weight - 92 000 hybrid plasminogen activator derived from human plasmin fibrin-binding and tissue plasminogen activator catalytic domains" * Abstract * | 1 | C 12 N 15/57 C 12 N 9/72 A 61 K 37/547 |
| | --- | | |
| A | WO-A-8 805 822 (AMERICAN HOME PRODUCTS) * Claims 1-4,14,15 * | 1 | |
| | --- | | |
| A | EP-A-0 293 934 (ZYMOGENETICS) * Claims; page 4, line 40 - page 5, line 17 * | 1,5,7,9 ,11-13 | |
| | ----- | | |

TECHNICAL FIELDS SEARCHED (Int. Cl.5)

C 12 N

The present search report has been drawn up for all claims

| Place of search | Date of completion of the search | Examiner |
|---|---|---|
| THE HAGUE | 30-03-1990 | HUBER A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)